(19) Europäisches Patentamt
European Patent Office
Office européen des brevets

(11) EP 3 875 607 A1

(12) EUROPEAN PATENT APPLICATION

(43) Date of publication:
**08.09.2021 Bulletin 2021/36**

(51) Int Cl.:
***C12Q 1/6886*** *(2018.01)*

(21) Application number: **20161129.0**

(22) Date of filing: **05.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **HOFFMANN, Ralf Dieter**
  **5656 AE Eindhoven (NL)**
- **ORSEL, Joukje Garrelina**
  **5656 AE Eindhoven (NL)**
- **VAN LIESHOUT, Ron Martinus Laurentius**
  **5656 AE Eindhoven (NL)**
- **DE KLERK - STARMANS, Maud**
  **5656 AE Eindhoven (NL)**

(74) Representative: **Philips Intellectual Property & Standards**
**High Tech Campus 5**
**5656 AE Eindhoven (NL)**

# (54) PREDICTION OF RADIOTHERAPY RESPONSE FOR PROSTATE CANCER SUBJECT BASED ON INTERLEUKIN GENES

(57) The invention relates to a method of predicting a response of a prostate cancer subject to radiotherapy, comprising determining or receiving the result of a determination of a gene expression profile for each of one or more interleukin genes selected from the group consisting of: IL17RE, IL1B, IL3, IL7R, IL9R, and EBI3, said gene expression profile(s) being determined in a biological sample obtained from the subject, and determining the prediction of the radiotherapy response based on the gene expression profile(s) for the one or more interleukin genes.

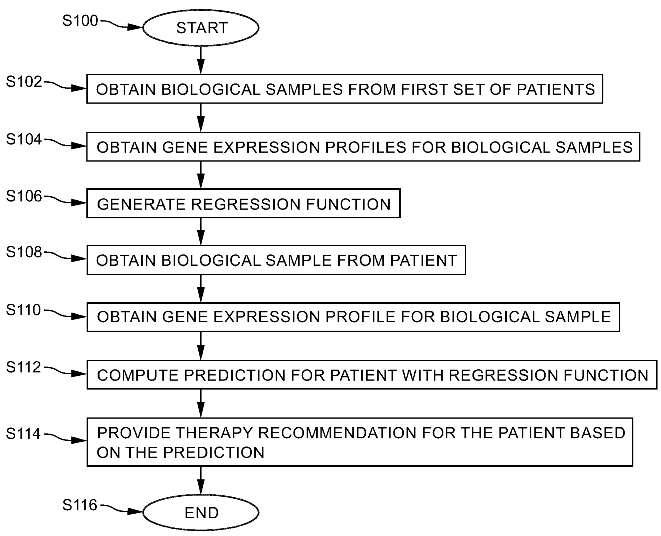


FIG. 1

EP 3 875 607 A1

SEQUENCE LISTING

<110> Koninklijke Philips N.V.

<120> Prediction of radiotherapy response for prostate cancer subject based on interleukin genes

<130> 2019PF00710

<160> 30

<170> PatentIn version 3.5

<210> 1
<211> 2004
<212> DNA
<213> Homo sapiens

<400> 1
atggggagct ccagactggc agccctgctc ctgcctctcc tcctcatagt catcgacctc 60

tctgactctg ctgggattgg ctttcgccac ctgccccact ggaacacccg ctgtcctctg 120

gcctcccaca cggatgacag tttcactgga agttctgcct atatcccttg ccgcacctgg 180

tgggccctct tctccacaaa gccttggtgt gtgcgagtct ggcactgttc ccgctgtttg 240

tgccagcatc tgctgtcagg tggctcaggt cttcaacggg gcctcttcca cctcctggtg 300

cagaaatcca aaaagtcttc cacattcaag ttctatagga gacacaagat gccagcacct 360

gctcagagga agctgctgcc tcgtcgtcac ctgtctgaga agagccatca catttccatc 420

ccctccccag acatctccca caagggactt cgctctaaaa ggacccaacc ttcggatcca 480

gagacatggg aaagtcttcc cagattggac tcacaaaggc atggaggacc cgagttctcc 540

tttgatttgc tgcctgaggc ccgggctatt cgggtgacca tatcttcagg ccctgaggtc 600

agcgtgcgtc tttgtcacca gtgggcactg gagtgtgaag agctgagcag tccctatgat 660

gtccagaaaa ttgtgtctgg gggccacact gtagagctgc cttatgaatt ccttctgccc 720

tgtctgtgca tagaggcatc ctacctgcaa gaggacactg tgaggcgcaa aaaatgtccc 780

ttccagagct ggccagaagc ctatggctcg gacttctgga agtcagtgca cttcactgac 840

tacagccagc acactcagat ggtcatggcc ctgacactcc gctgcccact gaagctggaa 900

gctgccctct gccagaggca cgactggcat accctttgca aagacctccc gaatgccaca 960

gctcgagagt cagatgggtg gtatgttttg gagaaggtgg acctgcaccc ccagctctgc 1020

ttcaagttct cttttggaaa cagcagccat gttgaatgcc cccaccagac tgggtctctc 1080

acatcctgga atgtaagcat ggatacccaa gcccagcagc tgattcttca cttctcctca 1140

agaatgcatg ccaccttcag tgctgcctgg agcctcccag gcttggggca ggacactttg 1200

gtgccccccg tgtacactgt cagccaggcc cggggctcaa gcccagtgtc actagacctc 1260

atcattccct tcctgaggcc agggtgctgt gtcctggtgt ggcggtcaga tgtccagttt 1320

gcctggaagc acctcttgtg tccggatgtc tcttacagac acctggggct cttgatcctg 1380

gcactgctgg ccctcctcac cctactgggt gttgttctgg ccctcacctg ccggcgccca 1440

cagtcaggcc cgggcccagc gcggccagtg ctcctcctgc acgcggcgga ctcggaggcg 1500

cagcggcgcc tggtgggagc gctggctgaa ctgctacggg cagcgctggg cggcgggcgc 1560

gacgtgatcg tggacctgtg ggaggggagg cacgtggcgc gcgtgggccc gctgccgtgg 1620

ctctgggcgg cgcggacgcg cgtagcgcgg gagcagggca ctgtgctgct gctgtggagc 1680

ggcgccgacc ttcgcccggt cagcggcccc gacccccgcg ccgcgcccct gctcgccctg 1740

ctccacgctg ccccgcgccc gctgctgctg ctcgcttact tcagtcgcct ctgcgccaag 1800

ggcgacatcc ccccgccgct gcgcgccctg ccgcgctacc gcctgctgcg cgacctgccg 1860

cgtctgctgc gggcgctgga cgcgcggcct ttcgcagagg ccaccagctg gggccgcctt 1920

ggggcgcggc agcgcaggca gagccgccta gagctgtgca gccggctcga acgagaggcc 1980

gcccgacttg cagacctagg ttga 2004

<210> 2
<211> 1602
<212> DNA
<213> Homo sapiens

<400> 2
atggggagct ccagactggc agccctgctc ctgcctctcc tcctcatagt catcgacctc 60

tctgactctg ctgggattgg ctttcgccac ctgccccact ggaacacccg ctgtcctctg 120

gcctcccaca cggatgacag tttcactgga agttctgcct atatcccttg ccgcacctgg 180

tgggccctct tctccacaaa gccttggtgt gtgcgagtct ggcactgttc ccgctgtttg 240

tgccagcatc tgctgtcagg tggctcaggt cttcaacggg gcctcttcca cctcctggtg 300

cagaaatcca aaaagtcttc cacattcaag ttctatagga gacacaagat gccagcacct 360

gctcagagga agctgctgcc tcgtcgtcac ctgtctgaga agagccatca catttccatc 420

ccctccccag acatctccca caagggactt cgctctaaaa ggacccaacc ttcggatcca 480

gagacatggg aaagtcttcc cagattggac tcacaaaggc atggaggacc cgagttctcc 540

tttgatttgc tgcctgaggc ccgggctatt cgggtgacca tatcttcagg ccctgaggtc 600

agcgtgcgtc tttgtcacca gtgggcactg gagtgtgaag agctgagcag tccctatgat 660

gtccagaaaa ttgtgtctgg gggccacact gtagagctgc cttatgaatt ccttctgccc 720

tgtctgtgca tagaggcatc ctacctgcaa gaggacactg tgaggcgcaa aaaatgtccc 780

ttccagagct ggccagaagc ctatggctcg gacttctgga agtcagtgca cttcactgac 840

tacagccagc acactcagat ggtcatggcc ctgacactcc gctgcccact gaagctggaa 900

gctgccctct gccagaggca cgactggcat accctttgca aagacctccc gaatgccaca 960

gctcgagagt cagatgggtg gtatgttttg gagaaggtgg acctgcaccc ccagctctgc 1020

ttcaagttct cttttggaaa cagcagccat gttgaatgcc cccaccagac tgggtctctc 1080

acatcctgga atgtaagcat ggatacccaa gcccagcagc tgattcttca cttctcctca 1140

agaatgcatg ccaccttcag tgctgcctgg agcctcccag gcttggggca ggacactttg 1200

gtgcccccg tgtacactgt cagccaggcc cggggctcaa gcccagtgtc actagacctc 1260

atcattccct tcctgaggcc agggtgctgt gtcctgctcc atgcttcact cagctccccg 1320

ggaggagaag atgcctggct catagggtg gggggctctg tgccctcagg tgtggcggtc 1380

agatgtccag tttgcctgga agcacctctt gtgtccggat gtctcttaca gacacctggg 1440

gctcttgatc ctggcactgc tggccctcct caccctactg ggtgttgttc tggccctcac 1500

ctgccggcgc ccacagtcag gcccgggccc agcgcggcca gtgctcctcc tgcacgcggc 1560

ggactcggag gcgcagcggc gcctggtggg agcgctggct ga 1602

<210> 3
<211> 667
<212> PRT
<213> Homo sapiens

<400> 3

Met Gly Ser Ser Arg Leu Ala Ala Leu Leu Leu Pro Leu Leu Leu Ile
1 5 10 15

Val Ile Asp Leu Ser Asp Ser Ala Gly Ile Gly Phe Arg His Leu Pro
20 25 30

His Trp Asn Thr Arg Cys Pro Leu Ala Ser His Thr Asp Asp Ser Phe
35 40 45

Thr Gly Ser Ser Ala Tyr Ile Pro Cys Arg Thr Trp Trp Ala Leu Phe
50 55 60

Ser Thr Lys Pro Trp Cys Val Arg Val Trp His Cys Ser Arg Cys Leu
65 70 75 80

Cys Gln His Leu Leu Ser Gly Gly Ser Gly Leu Gln Arg Gly Leu Phe
85 90 95

His Leu Leu Val Gln Lys Ser Lys Lys Ser Ser Thr Phe Lys Phe Tyr
100 105 110

Arg Arg His Lys Met Pro Ala Pro Ala Gln Arg Lys Leu Leu Pro Arg
115 120 125

Arg His Leu Ser Glu Lys Ser His His Ile Ser Ile Pro Ser Pro Asp

```
        130                 135                 140

Ile Ser His Lys Gly Leu Arg Ser Lys Arg Thr Gln Pro Ser Asp Pro
145                 150                 155                 160

Glu Thr Trp Glu Ser Leu Pro Arg Leu Asp Ser Gln Arg His Gly Gly
                165                 170                 175

Pro Glu Phe Ser Phe Asp Leu Leu Pro Glu Ala Arg Ala Ile Arg Val
            180                 185                 190

Thr Ile Ser Ser Gly Pro Glu Val Ser Val Arg Leu Cys His Gln Trp
        195                 200                 205

Ala Leu Glu Cys Glu Glu Leu Ser Ser Pro Tyr Asp Val Gln Lys Ile
    210                 215                 220

Val Ser Gly Gly His Thr Val Glu Leu Pro Tyr Glu Phe Leu Leu Pro
225                 230                 235                 240

Cys Leu Cys Ile Glu Ala Ser Tyr Leu Gln Glu Asp Thr Val Arg Arg
                245                 250                 255

Lys Lys Cys Pro Phe Gln Ser Trp Pro Glu Ala Tyr Gly Ser Asp Phe
            260                 265                 270

Trp Lys Ser Val His Phe Thr Asp Tyr Ser Gln His Thr Gln Met Val
        275                 280                 285

Met Ala Leu Thr Leu Arg Cys Pro Leu Lys Leu Glu Ala Ala Leu Cys
    290                 295                 300

Gln Arg His Asp Trp His Thr Leu Cys Lys Asp Leu Pro Asn Ala Thr
305                 310                 315                 320

Ala Arg Glu Ser Asp Gly Trp Tyr Val Leu Glu Lys Val Asp Leu His
                325                 330                 335

Pro Gln Leu Cys Phe Lys Phe Ser Phe Gly Asn Ser Ser His Val Glu
            340                 345                 350

Cys Pro His Gln Thr Gly Ser Leu Thr Ser Trp Asn Val Ser Met Asp
        355                 360                 365

Thr Gln Ala Gln Gln Leu Ile Leu His Phe Ser Ser Arg Met His Ala
    370                 375                 380
```

Thr Phe Ser Ala Ala Trp Ser Leu Pro Gly Leu Gly Gln Asp Thr Leu
385 390 395 400

Val Pro Pro Val Tyr Thr Val Ser Gln Ala Arg Gly Ser Ser Pro Val
405 410 415

Ser Leu Asp Leu Ile Ile Pro Phe Leu Arg Pro Gly Cys Cys Val Leu
420 425 430

Val Trp Arg Ser Asp Val Gln Phe Ala Trp Lys His Leu Leu Cys Pro
435 440 445

Asp Val Ser Tyr Arg His Leu Gly Leu Leu Ile Leu Ala Leu Leu Ala
450 455 460

Leu Leu Thr Leu Leu Gly Val Val Leu Ala Leu Thr Cys Arg Arg Pro
465 470 475 480

Gln Ser Gly Pro Gly Pro Ala Arg Pro Val Leu Leu Leu His Ala Ala
485 490 495

Asp Ser Glu Ala Gln Arg Arg Leu Val Gly Ala Leu Ala Glu Leu Leu
500 505 510

Arg Ala Ala Leu Gly Gly Gly Arg Asp Val Ile Val Asp Leu Trp Glu
515 520 525

Gly Arg His Val Ala Arg Val Gly Pro Leu Pro Trp Leu Trp Ala Ala
530 535 540

Arg Thr Arg Val Ala Arg Glu Gln Gly Thr Val Leu Leu Leu Trp Ser
545 550 555 560

Gly Ala Asp Leu Arg Pro Val Ser Gly Pro Asp Pro Arg Ala Ala Pro
565 570 575

Leu Leu Ala Leu Leu His Ala Ala Pro Arg Pro Leu Leu Leu Leu Ala
580 585 590

Tyr Phe Ser Arg Leu Cys Ala Lys Gly Asp Ile Pro Pro Pro Leu Arg
595 600 605

Ala Leu Pro Arg Tyr Arg Leu Leu Arg Asp Leu Pro Arg Leu Leu Arg
610 615 620

Ala Leu Asp Ala Arg Pro Phe Ala Glu Ala Thr Ser Trp Gly Arg Leu
625 630 635 640

```
Gly Ala Arg Gln Arg Arg Gln Ser Arg Leu Glu Leu Cys Ser Arg Leu
                645                 650                 655

Glu Arg Glu Ala Ala Arg Leu Ala Asp Leu Gly
            660                 665


<210>  4
<211>  533
<212>  PRT
<213>  Homo sapiens

<400>  4

Met Gly Ser Ser Arg Leu Ala Ala Leu Leu Leu Pro Leu Leu Leu Ile
1               5                   10                  15

Val Ile Asp Leu Ser Asp Ser Ala Gly Ile Gly Phe Arg His Leu Pro
            20                  25                  30

His Trp Asn Thr Arg Cys Pro Leu Ala Ser His Thr Asp Asp Ser Phe
        35                  40                  45

Thr Gly Ser Ser Ala Tyr Ile Pro Cys Arg Thr Trp Trp Ala Leu Phe
    50                  55                  60

Ser Thr Lys Pro Trp Cys Val Arg Val Trp His Cys Ser Arg Cys Leu
65                  70                  75                  80

Cys Gln His Leu Leu Ser Gly Gly Ser Gly Leu Gln Arg Gly Leu Phe
                85                  90                  95

His Leu Leu Val Gln Lys Ser Lys Lys Ser Ser Thr Phe Lys Phe Tyr
            100                 105                 110

Arg Arg His Lys Met Pro Ala Pro Ala Gln Arg Lys Leu Leu Pro Arg
        115                 120                 125

Arg His Leu Ser Glu Lys Ser His His Ile Ser Ile Pro Ser Pro Asp
    130                 135                 140

Ile Ser His Lys Gly Leu Arg Ser Lys Arg Thr Gln Pro Ser Asp Pro
145                 150                 155                 160

Glu Thr Trp Glu Ser Leu Pro Arg Leu Asp Ser Gln Arg His Gly Gly
                165                 170                 175

Pro Glu Phe Ser Phe Asp Leu Leu Pro Glu Ala Arg Ala Ile Arg Val
            180                 185                 190
```

```
Thr Ile Ser Ser Gly Pro Glu Val Ser Val Arg Leu Cys His Gln Trp
        195                 200                 205

Ala Leu Glu Cys Glu Glu Leu Ser Ser Pro Tyr Asp Val Gln Lys Ile
    210                 215                 220

Val Ser Gly Gly His Thr Val Glu Leu Pro Tyr Glu Phe Leu Leu Pro
225                 230                 235                 240

Cys Leu Cys Ile Glu Ala Ser Tyr Leu Gln Glu Asp Thr Val Arg Arg
                245                 250                 255

Lys Lys Cys Pro Phe Gln Ser Trp Pro Glu Ala Tyr Gly Ser Asp Phe
            260                 265                 270

Trp Lys Ser Val His Phe Thr Asp Tyr Ser Gln His Thr Gln Met Val
        275                 280                 285

Met Ala Leu Thr Leu Arg Cys Pro Leu Lys Leu Glu Ala Ala Leu Cys
    290                 295                 300

Gln Arg His Asp Trp His Thr Leu Cys Lys Asp Leu Pro Asn Ala Thr
305                 310                 315                 320

Ala Arg Glu Ser Asp Gly Trp Tyr Val Leu Glu Lys Val Asp Leu His
                325                 330                 335

Pro Gln Leu Cys Phe Lys Phe Ser Phe Gly Asn Ser Ser His Val Glu
            340                 345                 350

Cys Pro His Gln Thr Gly Ser Leu Thr Ser Trp Asn Val Ser Met Asp
        355                 360                 365

Thr Gln Ala Gln Gln Leu Ile Leu His Phe Ser Ser Arg Met His Ala
    370                 375                 380

Thr Phe Ser Ala Ala Trp Ser Leu Pro Gly Leu Gly Gln Asp Thr Leu
385                 390                 395                 400

Val Pro Pro Val Tyr Thr Val Ser Gln Ala Arg Gly Ser Ser Pro Val
                405                 410                 415

Ser Leu Asp Leu Ile Ile Pro Phe Leu Arg Pro Gly Cys Cys Val Leu
            420                 425                 430

Leu His Ala Ser Leu Ser Ser Pro Gly Gly Glu Asp Ala Trp Leu Ile
        435                 440                 445
```

Gly Val Gly Gly Ser Val Pro Ser Gly Val Ala Val Arg Cys Pro Val
    450                 455                 460

Cys Leu Glu Ala Pro Leu Val Ser Gly Cys Leu Leu Gln Thr Pro Gly
465                 470                 475                 480

Ala Leu Asp Pro Gly Thr Ala Gly Pro Pro His Pro Thr Gly Cys Cys
                485                 490                 495

Ser Gly Pro His Leu Pro Ala Pro Thr Val Arg Pro Gly Pro Ser Ala
            500                 505                 510

Ala Ser Ala Pro Pro Ala Arg Gly Gly Leu Gly Gly Ala Ala Ala Pro
        515                 520                 525

Gly Gly Ser Ala Gly
    530

<210>  5
<211>  191
<212>  PRT
<213>  Artificial Sequence

<220>
<223>  Artifical Sequence

<400>  5

Glu Met Gly Ser Ser Arg Leu Ala Ala Leu Leu Leu Pro Leu Leu Leu
1               5                   10                  15

Ile Val Ile Asp Leu Ser Asp Ser Ala Gly Ile Gly Phe Arg His Leu
            20                  25                  30

Pro His Trp Asn Thr Arg Cys Pro Leu Ala Ser His Thr Arg Lys Leu
        35                  40                  45

Leu Pro Arg Arg His Leu Ser Glu Lys Ser His His Ile Ser Ile Pro
    50                  55                  60

Ser Pro Asp Ile Ser His Lys Gly Leu Arg Ser Lys Arg Thr Gln Pro
65                  70                  75                  80

Ser Asp Pro Glu Thr Trp Glu Ser Leu Pro Arg Leu Asp Ser Gln Arg
                85                  90                  95

His Gly Gly Pro Glu Phe Ser Phe Asp Leu Leu Pro Glu Ala Arg Ala
            100                 105                 110

```
Ile Arg Val Thr Ile Ser Ser Gly Pro Glu Val Ser Val Arg Leu Cys
        115                 120                 125

His Gln Trp Ala Leu Glu Cys Glu Glu Leu Ser Ser Pro Tyr Asp Val
    130                 135                 140

Gln Lys Ile Val Ser Gly Gly His Thr Val Glu Leu Pro Tyr Glu Phe
145                 150                 155                 160

Leu Leu Pro Cys Leu Cys Ile Glu Met Ala Arg Thr Ser Gly Ser Gln
                165                 170                 175

Cys Thr Ser Leu Thr Thr Ala Ser Thr Leu Arg Trp Ser Trp Pro
            180                 185                 190


<210>  6
<211>  848
<212>  PRT
<213>  Artificial Sequence

<220>
<223>  Artificial Sequence

<400>  6

Glu Met Gly Ser Ser Arg Leu Ala Ala Leu Leu Leu Pro Leu Leu Leu
1               5                   10                  15

Ile Val Ile Asp Leu Ser Asp Ser Ala Gly Ile Gly Phe Arg His Leu
            20                  25                  30

Pro His Trp Asn Thr Arg Cys Pro Leu Ala Ser His Thr Val Phe Asn
        35                  40                  45

Gly Ala Ser Ser Thr Ser Trp Cys Arg Asn Pro Lys Ser Leu Pro His
    50                  55                  60

Ser Ser Ser Ile Gly Asp Thr Arg Cys Gln His Leu Leu Arg Gly Ser
65                  70                  75                  80

Cys Cys Leu Val Val Thr Cys Leu Arg Arg Ala Ile Thr Phe Pro Ser
                85                  90                  95

Pro Pro Gln Thr Ser Pro Thr Arg Asp Phe Ala Leu Lys Gly Pro Asn
            100                 105                 110

Leu Arg Ile Gln Arg His Gly Lys Val Phe Pro Asp Trp Thr His Lys
        115                 120                 125
```

Gly Met Glu Asp Pro Ser Ser Pro Leu Ile Cys Cys Leu Arg Pro Gly
    130                 135                 140

Leu Phe Gly Glu Met Leu Ala Pro Phe Leu Leu Leu Leu Thr His Ser
145                 150                 155                 160

Arg Ser Pro Leu Ser Phe Arg Pro Cys Ser His Val Pro Gly Ala Leu
                165                 170                 175

Ile Ala Gln Lys Pro Met Gly Ser Ser Arg Leu Ala Ala Leu Leu Leu
            180                 185                 190

Pro Leu Leu Leu Ile Val Ile Asp Leu Ser Asp Ser Ala Gly Ile Gly
        195                 200                 205

Phe Arg His Leu Pro His Trp Asn Thr Arg Cys Pro Leu Ala Ser His
    210                 215                 220

Thr Asp Asp Ser Phe Thr Gly Ser Ser Ala Tyr Ile Pro Cys Arg Thr
225                 230                 235                 240

Trp Trp Ala Leu Phe Ser Thr Lys Pro Trp Cys Val Arg Val Trp His
                245                 250                 255

Cys Ser Arg Cys Leu Cys Gln His Leu Leu Ser Gly Gly Ser Gly Leu
            260                 265                 270

Gln Arg Gly Leu Phe His Leu Leu Val Gln Lys Ser Lys Lys Ser Ser
        275                 280                 285

Thr Phe Lys Phe Tyr Arg Arg His Lys Met Pro Ala Pro Ala Gln Arg
    290                 295                 300

Lys Leu Leu Pro Arg Arg His Leu Ser Glu Lys Ser His His Ile Ser
305                 310                 315                 320

Ile Pro Ser Pro Asp Ile Ser His Lys Gly Leu Arg Ser Lys Arg Thr
                325                 330                 335

Gln Pro Ser Asp Pro Glu Thr Trp Glu Ser Leu Pro Arg Leu Asp Ser
            340                 345                 350

Gln Arg His Gly Gly Pro Glu Phe Ser Phe Asp Leu Leu Pro Glu Ala
        355                 360                 365

Arg Ala Ile Arg Val Thr Ile Ser Ser Gly Pro Glu Val Ser Val Arg
    370                 375                 380

Leu Cys His Gln Trp Ala Leu Glu Cys Glu Glu Leu Ser Ser Pro Tyr
385 390 395 400

Asp Val Gln Lys Ile Val Ser Gly Gly His Thr Val Glu Leu Pro Tyr
405 410 415

Glu Phe Leu Leu Pro Cys Leu Cys Ile Glu Ala Ser Tyr Leu Gln Glu
420 425 430

Asp Thr Val Arg Arg Lys Lys Cys Pro Phe Gln Ser Trp Pro Glu Ala
435 440 445

Tyr Gly Ser Asp Phe Trp Lys Ser Val His Phe Thr Asp Tyr Ser Gln
450 455 460

His Thr Gln Met Val Met Ala Leu Thr Leu Arg Cys Pro Leu Lys Leu
465 470 475 480

Glu Ala Ala Leu Cys Gln Arg His Asp Trp His Thr Leu Cys Lys Asp
485 490 495

Leu Pro Asn Ala Thr Ala Arg Glu Ser Asp Gly Trp Tyr Val Leu Glu
500 505 510

Lys Val Asp Leu His Pro Gln Leu Cys Phe Lys Phe Ser Phe Gly Asn
515 520 525

Ser Ser His Val Glu Cys Pro His Gln Thr Gly Ser Leu Thr Ser Trp
530 535 540

Asn Val Ser Met Asp Thr Gln Ala Gln Gln Leu Ile Leu His Phe Ser
545 550 555 560

Ser Arg Met His Ala Thr Phe Ser Ala Ala Trp Ser Leu Pro Gly Leu
565 570 575

Gly Gln Asp Thr Leu Val Pro Pro Val Tyr Thr Val Ser Gln Ala Arg
580 585 590

Gly Ser Ser Pro Val Ser Leu Asp Leu Ile Ile Pro Phe Leu Arg Pro
595 600 605

Gly Cys Cys Val Leu Val Trp Arg Ser Asp Val Gln Phe Ala Trp Lys
610 615 620

His Leu Leu Cys Pro Asp Val Ser Tyr Arg His Leu Gly Leu Leu Ile
625 630 635 640

Leu Ala Leu Leu Ala Leu Leu Thr Leu Leu Gly Val Val Leu Ala Leu
645 650 655

Thr Cys Arg Arg Pro Gln Ser Gly Pro Gly Pro Ala Arg Pro Val Leu
660 665 670

Leu Leu His Ala Ala Asp Ser Glu Ala Gln Arg Arg Leu Val Gly Ala
675 680 685

Leu Ala Glu Leu Leu Arg Ala Ala Leu Gly Gly Gly Arg Asp Val Ile
690 695 700

Val Asp Leu Trp Glu Gly Arg His Val Ala Arg Val Gly Pro Leu Pro
705 710 715 720

Trp Leu Trp Ala Ala Arg Thr Arg Val Ala Arg Glu Gln Gly Thr Val
725 730 735

Leu Leu Leu Trp Ser Gly Ala Asp Leu Arg Pro Val Ser Gly Pro Asp
740 745 750

Pro Arg Ala Ala Pro Leu Leu Ala Leu Leu His Ala Ala Pro Arg Pro
755 760 765

Leu Leu Leu Leu Ala Tyr Phe Ser Arg Leu Cys Ala Lys Gly Asp Ile
770 775 780

Pro Pro Pro Leu Arg Ala Leu Pro Arg Tyr Arg Leu Leu Arg Asp Leu
785 790 795 800

Pro Arg Leu Leu Arg Ala Leu Asp Ala Arg Pro Phe Ala Glu Ala Thr
805 810 815

Ser Trp Gly Arg Leu Gly Ala Arg Gln Arg Arg Gln Ser Arg Leu Glu
820 825 830

Leu Cys Ser Arg Leu Glu Arg Glu Ala Ala Arg Leu Ala Asp Leu Gly
835 840 845

<210> 7
<211> 701
<212> PRT
<213> Artificial Sequence

<220>
<223> Artificial Sequence

<400> 7

```
Glu Met Leu Ala Pro Phe Leu Leu Leu Leu Thr His Ser Arg Ser Pro
1               5                   10                  15

Leu Ser Phe Arg Pro Cys Ser His Val Pro Gly Ala Leu Ile Ala Gln
            20                  25                  30

Lys Pro Met Gly Ser Ser Arg Leu Ala Ala Leu Leu Leu Pro Leu Leu
        35                  40                  45

Leu Ile Val Ile Asp Leu Ser Asp Ser Ala Gly Ile Gly Phe Arg His
    50                  55                  60

Leu Pro His Trp Asn Thr Arg Cys Pro Leu Ala Ser His Thr Asp Asp
65                  70                  75                  80

Ser Phe Thr Gly Ser Ser Ala Tyr Ile Pro Cys Arg Thr Trp Trp Ala
                85                  90                  95

Leu Phe Ser Thr Lys Pro Trp Cys Val Arg Val Trp His Cys Ser Arg
            100                 105                 110

Cys Leu Cys Gln His Leu Leu Ser Gly Gly Ser Gly Leu Gln Arg Gly
        115                 120                 125

Leu Phe His Leu Leu Val Gln Lys Ser Lys Lys Ser Ser Thr Phe Lys
    130                 135                 140

Phe Tyr Arg Arg His Lys Met Pro Ala Pro Ala Gln Arg Lys Leu Leu
145                 150                 155                 160

Pro Arg Arg His Leu Ser Glu Lys Ser His His Ile Ser Ile Pro Ser
                165                 170                 175

Pro Asp Ile Ser His Lys Gly Leu Arg Ser Lys Arg Thr Gln Pro Ser
            180                 185                 190

Asp Pro Glu Thr Trp Glu Ser Leu Pro Arg Leu Asp Ser Gln Arg His
        195                 200                 205

Gly Gly Pro Glu Phe Ser Phe Asp Leu Leu Pro Glu Ala Arg Ala Ile
    210                 215                 220

Arg Val Thr Ile Ser Ser Gly Pro Glu Val Ser Val Arg Leu Cys His
225                 230                 235                 240

Gln Trp Ala Leu Glu Cys Glu Glu Leu Ser Ser Pro Tyr Asp Val Gln
                245                 250                 255
```

Lys Ile Val Ser Gly Gly His Thr Val Glu Leu Pro Tyr Glu Phe Leu
260 265 270

Leu Pro Cys Leu Cys Ile Glu Ala Ser Tyr Leu Gln Glu Asp Thr Val
275 280 285

Arg Arg Lys Lys Cys Pro Phe Gln Ser Trp Pro Glu Ala Tyr Gly Ser
290 295 300

Asp Phe Trp Lys Ser Val His Phe Thr Asp Tyr Ser Gln His Thr Gln
305 310 315 320

Met Val Met Ala Leu Thr Leu Arg Cys Pro Leu Lys Leu Glu Ala Ala
325 330 335

Leu Cys Gln Arg His Asp Trp His Thr Leu Cys Lys Asp Leu Pro Asn
340 345 350

Ala Thr Ala Arg Glu Ser Asp Gly Trp Tyr Val Leu Glu Lys Val Asp
355 360 365

Leu His Pro Gln Leu Cys Phe Lys Phe Ser Phe Gly Asn Ser Ser His
370 375 380

Val Glu Cys Pro His Gln Thr Gly Ser Leu Thr Ser Trp Asn Val Ser
385 390 395 400

Met Asp Thr Gln Ala Gln Gln Leu Ile Leu His Phe Ser Ser Arg Met
405 410 415

His Ala Thr Phe Ser Ala Ala Trp Ser Leu Pro Gly Leu Gly Gln Asp
420 425 430

Thr Leu Val Pro Pro Val Tyr Thr Val Ser Gln Ala Arg Gly Ser Ser
435 440 445

Pro Val Ser Leu Asp Leu Ile Ile Pro Phe Leu Arg Pro Gly Cys Cys
450 455 460

Val Leu Val Trp Arg Ser Asp Val Gln Phe Ala Trp Lys His Leu Leu
465 470 475 480

Cys Pro Asp Val Ser Tyr Arg His Leu Gly Leu Leu Ile Leu Ala Leu
485 490 495

Leu Ala Leu Leu Thr Leu Leu Gly Val Val Leu Ala Leu Thr Cys Arg

```
                500                 505                 510

Arg Pro Gln Ser Gly Pro Gly Pro Ala Arg Pro Val Leu Leu Leu His
            515                 520                 525

Ala Ala Asp Ser Glu Ala Gln Arg Arg Leu Val Gly Ala Leu Ala Glu
    530                 535                 540

Leu Leu Arg Ala Ala Leu Gly Gly Gly Arg Asp Val Ile Val Asp Leu
545                 550                 555                 560

Trp Glu Gly Arg His Val Ala Arg Val Gly Pro Leu Pro Trp Leu Trp
                565                 570                 575

Ala Ala Arg Thr Arg Val Ala Arg Glu Gln Gly Thr Val Leu Leu Leu
            580                 585                 590

Trp Ser Gly Ala Asp Leu Arg Pro Val Ser Gly Pro Asp Pro Arg Ala
        595                 600                 605

Ala Pro Leu Leu Ala Leu Leu His Ala Ala Pro Arg Pro Leu Leu Leu
    610                 615                 620

Leu Ala Tyr Phe Ser Arg Leu Cys Ala Lys Gly Asp Ile Pro Pro Pro
625                 630                 635                 640

Leu Arg Ala Leu Pro Arg Tyr Arg Leu Leu Arg Asp Leu Pro Arg Leu
                645                 650                 655

Leu Arg Ala Leu Asp Ala Arg Pro Phe Ala Glu Ala Thr Ser Trp Gly
            660                 665                 670

Arg Leu Gly Ala Arg Gln Arg Arg Gln Ser Arg Leu Glu Leu Cys Ser
        675                 680                 685

Arg Leu Glu Arg Glu Ala Ala Arg Leu Ala Asp Leu Gly
    690                 695                 700


<210>  8
<211>  174
<212>  PRT
<213>  Artificial Sequence

<220>
<223>  Artificial Sequence

<400>  8

Glu Met Gly Ser Ser Arg Leu Ala Ala Leu Leu Leu Pro Leu Leu Leu
1               5                   10                  15
```

```
Ile Val Ile Asp Leu Ser Asp Ser Ala Gly Ile Gly Phe Arg His Leu
            20                  25                  30

Pro His Trp Asn Thr Arg Cys Pro Leu Ala Ser His Thr Asp Asp Ser
        35                  40                  45

Phe Thr Gly Leu Gln Arg Gly Leu Phe His Leu Leu Val Gln Lys Ser
    50                  55                  60

Lys Lys Ser Ser Thr Phe Lys Phe Tyr Arg Arg His Lys Met Pro Ala
65                  70                  75                  80

Pro Ala Gln Arg Lys Leu Leu Pro Arg Arg His Leu Ser Glu Lys Ser
                85                  90                  95

His His Ile Ser Ile Pro Ser Pro Asp Ile Ser His Lys Gly Leu Arg
            100                 105                 110

Ser Lys Arg Thr Gln Pro Ser Asp Pro Glu Thr Trp Glu Ser Leu Pro
        115                 120                 125

Arg Leu Asp Ser Gln Arg His Gly Gly Pro Glu Phe Ser Phe Asp Leu
    130                 135                 140

Leu Pro Glu Ala Arg Ala Ile Arg Val Thr Ile Ser Ser Gly Pro Glu
145                 150                 155                 160

Val Ser Val Arg Leu Cys His Gln Trp Ala Leu Glu Cys Glu
                165                 170


<210>  9
<211>  187
<212>  PRT
<213>  Artificial Sequence

<220>
<223>  Artificial Sequence

<400>  9

Glu Met Gly Ser Ser Arg Leu Ala Ala Leu Leu Leu Pro Leu Leu Leu
1               5                   10                  15

Ile Val Ile Asp Leu Ser Asp Ser Ala Gly Ile Gly Phe Arg His Leu
            20                  25                  30

Pro His Trp Asn Thr Arg Cys Pro Leu Ala Ser His Thr Glu Val Leu
        35                  40                  45
```

```
Pro Ile Ser Leu Ala Ala Pro Gly Gly Pro Ser Ser Pro Gln Ser Leu
    50                  55                  60

Gly Val Cys Glu Ser Gly Thr Val Pro Ala Val Cys Ala Ser Ile Cys
65                  70                  75                  80

Cys Gln Val Ala Gln Val Phe Asn Gly Ala Ser Ser Thr Ser Trp Cys
                85                  90                  95

Arg Asn Pro Lys Ser Leu Pro His Ser Ser Ser Ile Gly Asp Thr Arg
            100                 105                 110

Cys Gln His Leu Leu Arg Gly Ser Cys Cys Leu Val Val Thr Cys Leu
        115                 120                 125

Arg Arg Ala Ile Thr Phe Pro Ser Pro Pro Gln Thr Ser Pro Thr Arg
    130                 135                 140

Asp Phe Ala Leu Lys Gly Pro Asn Leu Arg Ile Gln Arg His Gly Lys
145                 150                 155                 160

Val Phe Pro Asp Trp Thr His Lys Gly Met Glu Asp Pro Ser Ser Pro
                165                 170                 175

Leu Ile Cys Cys Leu Arg Pro Gly Leu Phe Gly
            180                 185
```

```
<210>  10
<211>  810
<212>  DNA
<213>  Homo sapiens

<400>  10
atggcagaag tacctgagct cgccagtgaa atgatggctt attacagtgg caatgaggat      60

gacttgttct ttgaagctga tggccctaaa cagatgaagt gctccttcca ggacctggac     120

ctctgccctc tggatggcgg catccagcta cgaatctccg accaccacta cagcaagggc     180

ttcaggcagg ccgcgtcagt tgttgtggcc atggacaagc tgaggaagat gctggttccc     240

tgcccacaga ccttccagga gaatgacctg agcaccttct ttcccttcat ctttgaagaa     300

gaacctatct tcttcgacac atgggataac gaggcttatg tgcacgatgc acctgtacga     360

tcactgaact gcacgctccg ggactcacag caaaaaagct tggtgatgtc tggtccatat     420

gaactgaaag ctctccacct ccagggacag gatatggagc aacaagtggt gttctccatg     480

tcctttgtac aaggagaaga aagtaatgac aaaatacctg tggccttggg cctcaaggaa     540

aagaatctgt acctgtcctg cgtgttgaaa gatgataagc ccactctaca gctggagagt     600
```

gtagatccca aaaattaccc aaagaagaag atggaaaagc gatttgtctt caacaagata 660

gaaatcaata acaagctgga atttgagtct gcccagttcc ccaactggta catcagcacc 720

tctcaagcag aaaacatgcc cgtcttcctg ggagggacca aaggcggcca ggatataact 780

gacttcacca tgcaatttgt gtcttcctaa 810

<210> 11
<211> 269
<212> PRT
<213> Homo sapiens

<400> 11

Met Ala Glu Val Pro Glu Leu Ala Ser Glu Met Met Ala Tyr Tyr Ser
1 5 10 15

Gly Asn Glu Asp Asp Leu Phe Phe Glu Ala Asp Gly Pro Lys Gln Met
20 25 30

Lys Cys Ser Phe Gln Asp Leu Asp Leu Cys Pro Leu Asp Gly Gly Ile
35 40 45

Gln Leu Arg Ile Ser Asp His His Tyr Ser Lys Gly Phe Arg Gln Ala
50 55 60

Ala Ser Val Val Val Ala Met Asp Lys Leu Arg Lys Met Leu Val Pro
65 70 75 80

Cys Pro Gln Thr Phe Gln Glu Asn Asp Leu Ser Thr Phe Phe Pro Phe
85 90 95

Ile Phe Glu Glu Glu Pro Ile Phe Phe Asp Thr Trp Asp Asn Glu Ala
100 105 110

Tyr Val His Asp Ala Pro Val Arg Ser Leu Asn Cys Thr Leu Arg Asp
115 120 125

Ser Gln Gln Lys Ser Leu Val Met Ser Gly Pro Tyr Glu Leu Lys Ala
130 135 140

Leu His Leu Gln Gly Gln Asp Met Glu Gln Gln Val Val Phe Ser Met
145 150 155 160

Ser Phe Val Gln Gly Glu Glu Ser Asn Asp Lys Ile Pro Val Ala Leu
165 170 175

Gly Leu Lys Glu Lys Asn Leu Tyr Leu Ser Cys Val Leu Lys Asp Asp
180 185 190

Lys Pro Thr Leu Gln Leu Glu Ser Val Asp Pro Lys Asn Tyr Pro Lys
195 200 205

Lys Lys Met Glu Lys Arg Phe Val Phe Asn Lys Ile Glu Ile Asn Asn
210 215 220

Lys Leu Glu Phe Glu Ser Ala Gln Phe Pro Asn Trp Tyr Ile Ser Thr
225 230 235 240

Ser Gln Ala Glu Asn Met Pro Val Phe Leu Gly Gly Thr Lys Gly Gly
245 250 255

Gln Asp Ile Thr Asp Phe Thr Met Gln Phe Val Ser Ser
260 265

<210> 12
<211> 125
<212> PRT
<213> Artificial Sequence

<220>
<223> Artificial Sequence

<400> 12

Met Ala Glu Val Pro Glu Leu Ala Ser Glu Met Met Ala Tyr Tyr Ser
1 5 10 15

Gly Asn Glu Asp Asp Leu Phe Phe Glu Ala Asp Gly Pro Lys Gln Met
20 25 30

Lys Cys Ser Phe Gln Asp Leu Asp Leu Cys Pro Leu Asp Gly Gly Ile
35 40 45

Gln Leu Arg Ile Ser Asp His His Tyr Ser Lys Gly Phe Arg Gln Ala
50 55 60

Ala Ser Val Val Val Ala Met Asp Lys Leu Arg Lys Met Leu Val Pro
65 70 75 80

Cys Pro Gln Thr Phe Gln Glu Asn Asp Leu Ser Thr Phe Phe Pro Phe
85 90 95

Ile Phe Glu Glu Glu Pro Ile Phe Phe Asp Thr Trp Asp Asn Glu Ala
100 105 110

Tyr Val His Asp Ala Pro Val Arg Ser Leu Asn Cys Thr
115 120 125

<210> 13
<211> 143
<212> PRT
<213> Artificial Sequence

<220>
<223> Artificial Sequence

<400> 13

Met Ala Glu Val Pro Glu Leu Ala Ser Glu Met Met Ala Tyr Tyr Ser
1 5 10 15

Gly Asn Glu Asp Asp Leu Phe Phe Glu Ala Asp Gly Pro Lys Gln Met
20 25 30

Lys Cys Ser Phe Gln Asp Leu Asp Leu Cys Pro Leu Asp Gly Gly Ile
35 40 45

Gln Leu Arg Ile Ser Asp His His Tyr Ser Lys Gly Phe Arg Gln Ala
50 55 60

Ala Ser Val Val Val Ala Met Asp Lys Leu Arg Lys Met Leu Val Pro
65 70 75 80

Cys Pro Gln Thr Phe Gln Glu Asn Asp Leu Ser Thr Phe Phe Pro Phe
85 90 95

Ile Phe Glu Glu Glu Pro Ile Phe Phe Asp Thr Trp Asp Asn Glu Ala
100 105 110

Tyr Val His Asp Ala Pro Val Arg Ser Leu Asn Cys Thr Leu Arg Asp
115 120 125

Ser Gln Gln Lys Ser Leu Val Met Ser Gly Pro Tyr Glu Leu Lys
130 135 140

<210> 14
<211> 111
<212> PRT
<213> Artificial Sequence

<220>
<223> Artificial Sequence

<400> 14

Met Ala Glu Val Pro Glu Leu Ala Ser Glu Met Met Ala Tyr Tyr Ser
1 5 10 15

Gly Asn Glu Asp Asp Leu Phe Phe Glu Ala Asp Gly Pro Lys Gln Met
20 25 30

Lys Cys Ser Phe Gln Asp Leu Asp Leu Cys Pro Leu Asp Gly Gly Ile
35 40 45

Gln Leu Arg Ile Ser Asp His His Tyr Ser Lys Gly Phe Arg Gln Ala
50 55 60

Ala Ser Val Val Val Ala Met Asp Lys Leu Arg Lys Met Leu Val Pro
65 70 75 80

Cys Pro Gln Thr Phe Gln Glu Asn Asp Leu Ser Thr Phe Phe Pro Phe
85 90 95

Ile Phe Glu Glu Glu Pro Ile Phe Phe Asp Thr Trp Asp Asn Glu
100 105 110

<210> 15
<211> 459
<212> DNA
<213> Homo sapiens

<400> 15
atgagccgcc tgcccgtcct gctcctgctc caactcctgg tccgccccgg actccaagct 60

cccatgaccc agacaacgcc cttgaagaca agctgggtta actgctctaa catgatcgat 120

gaaattataa cacacttaaa gcagccacct ttgcctttgc tggacttcaa caacctcaat 180

ggggaagacc aagacattct gatggaaaat aaccttcgaa ggccaaacct ggaggcattc 240

aacagggctg tcaagagttt acagaacgca tcagcaattg agagcattct taaaaatctc 300

ctgccatgtc tgcccctggc cacggccgca cccacgcgac atccaatcca tatcaaggac 360

ggtgactgga atgaattccg gaggaaactg acgttctatc tgaaaaccct tgagaatgcg 420

caggctcaac agacgacttt gagcctcgcg atcttttga 459

<210> 16
<211> 152
<212> PRT
<213> Homo sapiens

<400> 16

Met Ser Arg Leu Pro Val Leu Leu Leu Leu Gln Leu Leu Val Arg Pro
1 5 10 15

Gly Leu Gln Ala Pro Met Thr Gln Thr Thr Pro Leu Lys Thr Ser Trp
20 25 30

Val Asn Cys Ser Asn Met Ile Asp Glu Ile Ile Thr His Leu Lys Gln
35 40 45

Pro Pro Leu Pro Leu Leu Asp Phe Asn Asn Leu Asn Gly Glu Asp Gln
50 55 60

Asp Ile Leu Met Glu Asn Asn Leu Arg Arg Pro Asn Leu Glu Ala Phe
65 70 75 80

Asn Arg Ala Val Lys Ser Leu Gln Asn Ala Ser Ala Ile Glu Ser Ile
85 90 95

Leu Lys Asn Leu Leu Pro Cys Leu Pro Leu Ala Thr Ala Ala Pro Thr
100 105 110

Arg His Pro Ile His Ile Lys Asp Gly Asp Trp Asn Glu Phe Arg Arg
115 120 125

Lys Leu Thr Phe Tyr Leu Lys Thr Leu Glu Asn Ala Gln Ala Gln Gln
130 135 140

Thr Thr Leu Ser Leu Ala Ile Phe
145 150

<210> 17
<211> 1380
<212> DNA
<213> Homo sapiens

<400> 17
atgacaattc taggtacaac ttttggcatg gtttttctt tacttcaagt cgtttctgga 60

gaaagtggct atgctcaaaa tggagacttg gaagatgcag aactggatga ctactcattc 120

tcatgctata gccagttgga agtgaatgga tcgcagcact cactgacctg tgcttttgag 180

gacccagatg tcaacatcac caatctggaa tttgaaatat gtggggccct cgtggaggta 240

aagtgcctga atttcaggaa actacaagag atatatttca tcgagacaaa gaaattctta 300

ctgattggaa agagcaatat atgtgtgaag gttggagaaa agagtctaac ctgcaaaaaa 360

atagacctaa ccactatagt taaacctgag gctccttttg acctgagtgt cgtctatcgg 420

gaaggagcca atgactttgt ggtgacattt aatacatcac acttgcaaaa gaagtatgta 480

aaagttttaa tgcacgatgt agcttaccgc caggaaaagg atgaaaacaa atggacgcat 540

gtgaatttat ccagcacaaa gctgacactc ctgcagagaa agctccaacc ggcagcaatg 600

tatgagatta aagttcgatc catccctgat cactatttta aaggcttctg gagtgaatgg 660

agtccaagtt attacttcag aactccagag atcaataata gctcagggga gatggatcct 720

atcttactaa ccatcagcat tttgagtttt ttctctgtcg ctctgttggt catcttggcc 780

tgtgtgttat ggaaaaaaag gattaagcct atcgtatggc ccagtctccc cgatcataag 840

aagactctgg aacatctttg taagaaacca agaaaaaatt taaatgtgag tttcaatcct 900

```
gaaagtttcc tggactgcca gattcatagg gtggatgaca ttcaagctag agatgaagtg      960

gaaggttttc tgcaagatac gtttcctcag caactagaag aatctgagaa gcagaggctt     1020

ggaggggatg tgcagagccc caactgccca tctgaggatg tagtcatcac tccagaaagc     1080

tttggaagag attcatccct cacatgcctg gctgggaatg tcagtgcatg tgacgcccct     1140

attctctcct cttccaggtc cctagactgc agggagagtg gcaagaatgg gcctcatgtg     1200

taccaggacc tcctgcttag ccttgggact acaaacagca cgctgccccc tccattttct     1260

ctccaatctg gaatcctgac attgaaccca gttgctcagg gtcagcccat tcttacttcc     1320

ctgggatcaa atcaagaaga agcatatgtc accatgtcca gcttctacca aaaccagtga     1380
```

```
<210>  18
<211>  459
<212>  PRT
<213>  Homo sapiens

<400>  18

Met Thr Ile Leu Gly Thr Thr Phe Gly Met Val Phe Ser Leu Leu Gln
1               5                   10                  15

Val Val Ser Gly Glu Ser Gly Tyr Ala Gln Asn Gly Asp Leu Glu Asp
            20                  25                  30

Ala Glu Leu Asp Asp Tyr Ser Phe Ser Cys Tyr Ser Gln Leu Glu Val
        35                  40                  45

Asn Gly Ser Gln His Ser Leu Thr Cys Ala Phe Glu Asp Pro Asp Val
    50                  55                  60

Asn Ile Thr Asn Leu Glu Phe Glu Ile Cys Gly Ala Leu Val Glu Val
65                  70                  75                  80

Lys Cys Leu Asn Phe Arg Lys Leu Gln Glu Ile Tyr Phe Ile Glu Thr
                85                  90                  95

Lys Lys Phe Leu Leu Ile Gly Lys Ser Asn Ile Cys Val Lys Val Gly
            100                 105                 110

Glu Lys Ser Leu Thr Cys Lys Lys Ile Asp Leu Thr Thr Ile Val Lys
        115                 120                 125

Pro Glu Ala Pro Phe Asp Leu Ser Val Val Tyr Arg Glu Gly Ala Asn
    130                 135                 140

Asp Phe Val Val Thr Phe Asn Thr Ser His Leu Gln Lys Lys Tyr Val
145                 150                 155                 160
```

```
Lys Val Leu Met His Asp Val Ala Tyr Arg Gln Glu Lys Asp Glu Asn
                165                 170                 175

Lys Trp Thr His Val Asn Leu Ser Ser Thr Lys Leu Thr Leu Leu Gln
            180                 185                 190

Arg Lys Leu Gln Pro Ala Ala Met Tyr Glu Ile Lys Val Arg Ser Ile
        195                 200                 205

Pro Asp His Tyr Phe Lys Gly Phe Trp Ser Glu Trp Ser Pro Ser Tyr
    210                 215                 220

Tyr Phe Arg Thr Pro Glu Ile Asn Asn Ser Ser Gly Glu Met Asp Pro
225                 230                 235                 240

Ile Leu Leu Thr Ile Ser Ile Leu Ser Phe Phe Ser Val Ala Leu Leu
                245                 250                 255

Val Ile Leu Ala Cys Val Leu Trp Lys Lys Arg Ile Lys Pro Ile Val
            260                 265                 270

Trp Pro Ser Leu Pro Asp His Lys Lys Thr Leu Glu His Leu Cys Lys
        275                 280                 285

Lys Pro Arg Lys Asn Leu Asn Val Ser Phe Asn Pro Glu Ser Phe Leu
    290                 295                 300

Asp Cys Gln Ile His Arg Val Asp Asp Ile Gln Ala Arg Asp Glu Val
305                 310                 315                 320

Glu Gly Phe Leu Gln Asp Thr Phe Pro Gln Gln Leu Glu Glu Ser Glu
                325                 330                 335

Lys Gln Arg Leu Gly Gly Asp Val Gln Ser Pro Asn Cys Pro Ser Glu
            340                 345                 350

Asp Val Val Ile Thr Pro Glu Ser Phe Gly Arg Asp Ser Ser Leu Thr
        355                 360                 365

Cys Leu Ala Gly Asn Val Ser Ala Cys Asp Ala Pro Ile Leu Ser Ser
    370                 375                 380

Ser Arg Ser Leu Asp Cys Arg Glu Ser Gly Lys Asn Gly Pro His Val
385                 390                 395                 400

Tyr Gln Asp Leu Leu Leu Ser Leu Gly Thr Thr Asn Ser Thr Leu Pro
```

405 410 415

Pro Pro Phe Ser Leu Gln Ser Gly Ile Leu Thr Leu Asn Pro Val Ala
420 425 430

Gln Gly Gln Pro Ile Leu Thr Ser Leu Gly Ser Asn Gln Glu Glu Ala
435 440 445

Tyr Val Thr Met Ser Ser Phe Tyr Gln Asn Gln
450 455

<210> 19
<211> 459
<212> PRT
<213> Artificial Sequence

<220>
<223> Artificial Sequence

<400> 19

Met Thr Ile Leu Gly Thr Thr Phe Gly Met Val Phe Ser Leu Leu Gln
1 5 10 15

Val Val Ser Gly Glu Ser Gly Tyr Ala Gln Asn Gly Asp Leu Glu Asp
20 25 30

Ala Glu Leu Asp Asp Tyr Ser Phe Ser Cys Tyr Ser Gln Leu Glu Val
35 40 45

Asn Gly Ser Gln His Ser Leu Thr Cys Ala Phe Glu Asp Pro Asp Val
50 55 60

Asn Ile Thr Asn Leu Glu Phe Glu Ile Cys Gly Ala Leu Val Glu Val
65 70 75 80

Lys Cys Leu Asn Phe Arg Lys Leu Gln Glu Ile Tyr Phe Ile Glu Thr
85 90 95

Lys Lys Phe Leu Leu Ile Gly Lys Ser Asn Ile Cys Val Lys Val Gly
100 105 110

Glu Lys Ser Leu Thr Cys Lys Lys Ile Asp Leu Thr Thr Ile Val Lys
115 120 125

Pro Glu Ala Pro Phe Asp Leu Ser Val Val Tyr Arg Glu Gly Ala Asn
130 135 140

Asp Phe Val Val Thr Phe Asn Thr Ser His Leu Gln Lys Lys Tyr Val
145 150 155 160

Lys Val Leu Met His Asp Val Ala Tyr Arg Gln Glu Lys Asp Glu Asn
165 170 175

Lys Trp Thr His Val Asn Leu Ser Ser Thr Lys Leu Thr Leu Leu Gln
180 185 190

Arg Lys Leu Gln Pro Ala Ala Met Tyr Glu Ile Lys Val Arg Ser Ile
195 200 205

Pro Asp His Tyr Phe Lys Gly Phe Trp Ser Glu Trp Ser Pro Ser Tyr
210 215 220

Tyr Phe Arg Thr Pro Glu Ile Asn Asn Ser Ser Gly Glu Met Asp Pro
225 230 235 240

Ile Leu Leu Thr Ile Ser Ile Leu Ser Phe Phe Ser Val Ala Leu Leu
245 250 255

Val Ile Leu Ala Cys Val Leu Trp Lys Lys Arg Ile Lys Pro Ile Val
260 265 270

Trp Pro Ser Leu Pro Asp His Lys Lys Thr Leu Glu His Leu Cys Lys
275 280 285

Lys Pro Arg Lys Asn Leu Asn Val Ser Phe Asn Pro Glu Ser Phe Leu
290 295 300

Asp Cys Gln Ile His Arg Val Asp Asp Ile Gln Ala Arg Asp Glu Val
305 310 315 320

Glu Gly Phe Leu Gln Asp Thr Phe Pro Gln Gln Leu Glu Glu Ser Glu
325 330 335

Lys Gln Arg Leu Gly Gly Asp Val Gln Ser Pro Asn Cys Pro Ser Glu
340 345 350

Asp Val Val Ile Thr Pro Glu Ser Phe Gly Arg Asp Ser Ser Leu Thr
355 360 365

Cys Leu Ala Gly Asn Val Ser Ala Cys Asp Ala Pro Ile Leu Ser Ser
370 375 380

Ser Arg Ser Leu Asp Cys Arg Glu Ser Gly Lys Asn Gly Pro His Val
385 390 395 400

Tyr Gln Asp Leu Leu Leu Ser Leu Gly Thr Thr Asn Ser Thr Leu Pro

405 410 415

Pro Pro Phe Ser Leu Gln Ser Gly Ile Leu Thr Leu Asn Pro Val Ala
420 425 430

Gln Gly Gln Pro Ile Leu Thr Ser Leu Gly Ser Asn Gln Glu Glu Ala
435 440 445

Tyr Val Thr Met Ser Ser Phe Tyr Gln Asn Gln
450 455

<210> 20
<211> 52
<212> PRT
<213> Artificial Sequence

<220>
<223> Artificial Sequence

<400> 20

Met Thr Ile Leu Gly Thr Thr Phe Gly Met Val Phe Ser Leu Leu Gln
1 5 10 15

Val Val Ser Gly Glu Ser Gly Tyr Ala Gln Asn Gly Asp Leu Glu Asp
20 25 30

Ala Glu Leu Asp Asp Tyr Ser Phe Ser Cys Tyr Ser Gln Leu Glu Val
35 40 45

Asn Gly Ser Gln
50

<210> 21
<211> 180
<212> PRT
<213> Artificial Sequence

<220>
<223> Artificial Sequence

<400> 21

Met Thr Ile Leu Gly Thr Thr Phe Gly Met Val Phe Ser Leu Leu Gln
1 5 10 15

Val Val Ser Gly Glu Ser Gly Tyr Ala Gln Asn Gly Asp Leu Glu Asp
20 25 30

Ala Glu Leu Asp Asp Tyr Ser Phe Ser Cys Tyr Ser Gln Leu Glu Val
35 40 45

```
Asn Gly Ser Gln His Ser Leu Thr Cys Ala Phe Glu Asp Pro Asp Val
    50                  55                  60

Asn Ile Thr Asn Leu Glu Phe Glu Ile Cys Gly Ala Leu Val Glu Val
65                  70                  75                  80

Lys Cys Leu Asn Phe Arg Lys Leu Gln Glu Ile Tyr Phe Ile Glu Thr
                85                  90                  95

Lys Lys Phe Leu Leu Ile Gly Lys Ser Asn Ile Cys Val Lys Val Gly
            100                 105                 110

Glu Lys Ser Leu Thr Cys Lys Lys Ile Asp Leu Thr Thr Ile Val Lys
        115                 120                 125

Pro Glu Ala Pro Phe Asp Leu Ser Val Val Tyr Arg Glu Gly Ala Asn
    130                 135                 140

Asp Phe Val Val Thr Phe Asn Thr Ser His Leu Gln Lys Lys Tyr Val
145                 150                 155                 160

Lys Val Leu Met His Asp Val Ala Tyr Arg Gln Glu Lys Asp Glu Asn
                165                 170                 175

Lys Trp Thr Asp
            180


<210>  22
<211>  150
<212>  PRT
<213>  Artificial Sequence

<220>
<223>  Artificial Sequence

<400>  22

Met Thr Ile Leu Gly Thr Thr Phe Gly Met Val Phe Ser Leu Leu Gln
1               5                   10                  15

Val Val Ser Gly Glu Ser Gly Tyr Ala Gln Asn Gly Asp Leu Glu Asp
            20                  25                  30

Ala Glu Leu Asp Asp Tyr Ser Phe Ser Cys Tyr Ser Gln Leu Glu Val
        35                  40                  45

Asn Gly Ser Gln His Ser Leu Thr Cys Ala Phe Glu Asp Pro Asp Val
    50                  55                  60

Asn Ile Thr Asn Leu Glu Phe Glu Ile Cys Gly Ala Leu Val Glu Val
```

65 70 75 80

Lys Cys Leu Asn Phe Arg Lys Leu Gln Glu Ile Tyr Phe Ile Glu Thr
85 90 95

Lys Lys Phe Leu Leu Ile Gly Lys Ser Asn Ile Cys Val Lys Val Gly
100 105 110

Glu Lys Ser Leu Thr Cys Lys Lys Ile Asp Leu Thr Thr Ile Gly Lys
115 120 125

Lys Leu Tyr Ile Lys Val Trp Leu Ser Leu Leu Gly Tyr Leu Lys Thr
130 135 140

Leu Cys Leu Asp Ile Leu
145 150

<210> 23
<211> 59
<212> PRT
<213> Artificial Sequence

<220>
<223> Artificial Sequence

<400> 23

Met Thr Ile Leu Gly Thr Thr Phe Gly Met Val Phe Ser Leu Leu Gln
1 5 10 15

Val Val Ser Gly Glu Ser Gly Tyr Ala Gln Asn Gly Asp Leu Glu Asp
20 25 30

Ala Glu Leu Asp Asp Tyr Ser Phe Ser Cys Tyr Ser Gln Leu Glu Val
35 40 45

Asn Gly Ser Gln His Ser Leu Thr Cys Ala Phe
50 55

<210> 24
<211> 65
<212> PRT
<213> Artificial Sequence

<220>
<223> Artificial Sequence

<400> 24

Ala Ala Met Tyr Glu Ile Lys Val Arg Ser Ile Pro Asp His Tyr Phe
1 5 10 15

Lys Gly Phe Trp Ser Glu Trp Ser Pro Ser Tyr Tyr Phe Arg Thr Pro
20 25 30

Glu Ile Asn Asn Ser Ser Gly Leu Ser Leu Ser Tyr Gly Pro Val Ser
35 40 45

Pro Ile Ile Arg Arg Leu Trp Asn Ile Phe Val Arg Asn Gln Glu Lys
50 55 60

Ile
65

<210> 25
<211> 1029
<212> DNA
<213> Homo sapiens

<400> 25
atgcctcaga cttgtgatgg gactgggcag atgcatctgg gaagtaactg ctgcaagaac 60

ggacagacac tgctgcagag aacttgccac ggtgtttcat gctgtggctg gtggttccag 120

gctgcacgct ccattctagg aaaggggccc tcagcccagt cccttgcagg ctggaccttg 180

gagagtgagg ccctgaggcg agacatgggc acctggctcc tggcctgcat ctgcatctgc 240

acctgtgtct gcttgggagt ctctgtcaca ggggaaggac aagggccaag gtctagaacc 300

ttcacctgcc tcaccaacaa cattctcagg atcgattgcc actggtctgc cccagagctg 360

ggacagggct ccagcccctg gctcctcttc accaggctcc tggcggcaca cataagtgca 420

tcttgcgggg cagtgagtgc accgtcgtgc tgccacctga ggcagtgctc gtgccatctg 480

acaatttcac catcactttc caccactgca tgtctgggag ggagcaggtc agcctggtgg 540

acccggagta cctgccccgg agacacgagc aacatcagtt ctggccactg catcctgacc 600

tggagcatca gtcctgcctt ggagccaatg accacacttc tcagctatga gctggccttc 660

aagaagcagg aagaggcctg ggagcaggcc cagcacaggg atcacattgt cggggtgacc 720

tggcttatac ttgaagcctt tgagctggac cctggcttta tccatgaggc caggctgcgt 780

gtccagatgg ccacactgga ggatgatgtg gtagaggagg agcgttatac aggccagtgg 840

agtgagtgga gccagcctgt gtgcttccag gctccccaga gacaaggccc tctgatccca 900

ccctgggggt ggccaggcaa cacccttgtt gctgtgtcca tctttctcct gctgactggc 960

ccgacctacc tcctgttcaa gctgtcgccc agacttggat gggggcccac ggggccggtg 1020

tgctgttga 1029

<210> 26
<211> 1566
<212> DNA
<213> Homo sapiens

<400> 26
atgggactgg gcagatgcat ctgggaaggc tggaccttgg agagtgaggc cctgaggcga 60

gacatgggca cctggctcct ggcctgcatc tgcatctgca cctgtgtctg cttgggagtc 120

tctgtcacag gggaaggaca agggccaagg tctagaacct tcacctgcct caccaacaac 180

attctcagga tcgattgcca ctggtctgcc ccagagctgg gacagggctc cagcccctgg 240

ctcctcttca ccagcaacca ggctcctggc ggcacacata agtgcatctt gcggggcagt 300

gagtgcaccg tcgtgctgcc acctgaggca gtgctcgtgc catctgacaa tttcaccatc 360

actttccacc actgcatgtc tgggagggag caggtcagcc tggtggaccc ggagtacctg 420

ccccggagac acgttaagct ggacccgccc tctgacttgc agagcaacat cagttctggc 480

cactgcatcc tgacctggag catcagtcct gccttggagc caatgaccac acttctcagc 540

tatgagctgg ccttcaagaa gcaggaagag gcctgggagc aggcccagca cagggatcac 600

attgtcgggg tgacctggct tatacttgaa gcctttgagc tggaccctgg ctttatccat 660

gaggccaggc tgcgtgtcca gatggccaca ctggaggatg atgtggtaga ggaggagcgt 720

tatacaggcc agtggagtga gtggagccag cctgtgtgct tccaggctcc ccagagacaa 780

ggccctctga tcccaccctg gggtggccca ggcaacaccc ttgttgctgt gtccatcttt 840

ctcctgctga ctggcccgac ctacctcctg ttcaagctgt cgcccagggt gaagagaatc 900

ttctaccaga acgtgccctc tccagcgatg ttcttccagc ccctctacag tgtacacaat 960

gggaacttcc agacttggat gggggcccac ggggccggtg tgctgttgag ccaggactgt 1020

gctggcaccc cacagggagc cttggagccc tgcgtccagg aggccactgc actgctcact 1080

tgtggcccag cgcgtccttg gaaatctgtg gccctggagg aggaacagga gggccctggg 1140

accaggctcc cggggaacct gagctcagag gatgtgctgc cagcagggtg tacggagtgg 1200

agggtacaga cgcttgccta tctgccacag gaggactggg cccccacgtc cctgactagg 1260

ccggctcccc cagactcaga gggcagcagg agcagcagca gcagcagcag cagcaacaac 1320

aacaactact gtgccttggg ctgctatggg ggatggcacc tctcagccct cccaggaaac 1380

acacagagct ctgggcccat cccagccctg gcctgtggcc tttcttgtga ccatcagggc 1440

ctggagaccc agcaaggagt tgcctgggtg ctggctggtc actgccagag gcctgggctg 1500

catgaggacc tccagggcat gttgctccct tctgtcctca gcaaggctcg gtcctggaca 1560

ttctag 1566

<210> 27
<211> 342
<212> PRT
<213> Homo sapiens

<400> 27

```
Met Pro Gln Thr Cys Asp Gly Thr Gly Gln Met His Leu Gly Ser Asn
1               5                   10                  15

Cys Cys Lys Asn Gly Gln Thr Leu Leu Gln Arg Thr Cys His Gly Val
            20                  25                  30

Ser Cys Cys Gly Trp Trp Phe Gln Ala Ala Arg Ser Ile Leu Gly Lys
        35                  40                  45

Gly Pro Ser Ala Gln Ser Leu Ala Gly Trp Thr Leu Glu Ser Glu Ala
    50                  55                  60

Leu Arg Arg Asp Met Gly Thr Trp Leu Leu Ala Cys Ile Cys Ile Cys
65                  70                  75                  80

Thr Cys Val Cys Leu Gly Val Ser Val Thr Gly Glu Gly Gln Gly Pro
                85                  90                  95

Arg Ser Arg Thr Phe Thr Cys Leu Thr Asn Asn Ile Leu Arg Ile Asp
            100                 105                 110

Cys His Trp Ser Ala Pro Glu Leu Gly Gln Gly Ser Ser Pro Trp Leu
        115                 120                 125

Leu Phe Thr Arg Leu Leu Ala Ala His Ile Ser Ala Ser Cys Gly Ala
    130                 135                 140

Val Ser Ala Pro Ser Cys Cys His Leu Arg Gln Cys Ser Cys His Leu
145                 150                 155                 160

Thr Ile Ser Pro Ser Leu Ser Thr Thr Ala Cys Leu Gly Gly Ser Arg
                165                 170                 175

Ser Ala Trp Trp Thr Arg Ser Thr Cys Pro Gly Asp Thr Ser Asn Ile
            180                 185                 190

Ser Ser Gly His Cys Ile Leu Thr Trp Ser Ile Ser Pro Ala Leu Glu
        195                 200                 205

Pro Met Thr Thr Leu Leu Ser Tyr Glu Leu Ala Phe Lys Lys Gln Glu
    210                 215                 220

Glu Ala Trp Glu Gln Ala Gln His Arg Asp His Ile Val Gly Val Thr
225                 230                 235                 240

Trp Leu Ile Leu Glu Ala Phe Glu Leu Asp Pro Gly Phe Ile His Glu
                245                 250                 255
```

```
Ala Arg Leu Arg Val Gln Met Ala Thr Leu Glu Asp Asp Val Val Glu
            260                 265                 270

Glu Glu Arg Tyr Thr Gly Gln Trp Ser Glu Trp Ser Gln Pro Val Cys
        275                 280                 285

Phe Gln Ala Pro Gln Arg Gln Gly Pro Leu Ile Pro Pro Trp Gly Trp
    290                 295                 300

Pro Gly Asn Thr Leu Val Ala Val Ser Ile Phe Leu Leu Leu Thr Gly
305                 310                 315                 320

Pro Thr Tyr Leu Leu Phe Lys Leu Ser Pro Arg Leu Gly Trp Gly Pro
                325                 330                 335

Thr Gly Pro Val Cys Cys
            340


<210>  28
<211>  521
<212>  PRT
<213>  Homo sapiens

<400>  28

Met Gly Leu Gly Arg Cys Ile Trp Glu Gly Trp Thr Leu Glu Ser Glu
1               5                   10                  15

Ala Leu Arg Arg Asp Met Gly Thr Trp Leu Leu Ala Cys Ile Cys Ile
            20                  25                  30

Cys Thr Cys Val Cys Leu Gly Val Ser Val Thr Gly Glu Gly Gln Gly
        35                  40                  45

Pro Arg Ser Arg Thr Phe Thr Cys Leu Thr Asn Asn Ile Leu Arg Ile
    50                  55                  60

Asp Cys His Trp Ser Ala Pro Glu Leu Gly Gln Gly Ser Ser Pro Trp
65                  70                  75                  80

Leu Leu Phe Thr Ser Asn Gln Ala Pro Gly Gly Thr His Lys Cys Ile
                85                  90                  95

Leu Arg Gly Ser Glu Cys Thr Val Val Leu Pro Pro Glu Ala Val Leu
            100                 105                 110

Val Pro Ser Asp Asn Phe Thr Ile Thr Phe His His Cys Met Ser Gly
        115                 120                 125
```

Arg Glu Gln Val Ser Leu Val Asp Pro Glu Tyr Leu Pro Arg Arg His
130 135 140

Val Lys Leu Asp Pro Pro Ser Asp Leu Gln Ser Asn Ile Ser Ser Gly
145 150 155 160

His Cys Ile Leu Thr Trp Ser Ile Ser Pro Ala Leu Glu Pro Met Thr
165 170 175

Thr Leu Leu Ser Tyr Glu Leu Ala Phe Lys Lys Gln Glu Glu Ala Trp
180 185 190

Glu Gln Ala Gln His Arg Asp His Ile Val Gly Val Thr Trp Leu Ile
195 200 205

Leu Glu Ala Phe Glu Leu Asp Pro Gly Phe Ile His Glu Ala Arg Leu
210 215 220

Arg Val Gln Met Ala Thr Leu Glu Asp Asp Val Val Glu Glu Glu Arg
225 230 235 240

Tyr Thr Gly Gln Trp Ser Glu Trp Ser Gln Pro Val Cys Phe Gln Ala
245 250 255

Pro Gln Arg Gln Gly Pro Leu Ile Pro Pro Trp Gly Trp Pro Gly Asn
260 265 270

Thr Leu Val Ala Val Ser Ile Phe Leu Leu Leu Thr Gly Pro Thr Tyr
275 280 285

Leu Leu Phe Lys Leu Ser Pro Arg Val Lys Arg Ile Phe Tyr Gln Asn
290 295 300

Val Pro Ser Pro Ala Met Phe Phe Gln Pro Leu Tyr Ser Val His Asn
305 310 315 320

Gly Asn Phe Gln Thr Trp Met Gly Ala His Gly Ala Gly Val Leu Leu
325 330 335

Ser Gln Asp Cys Ala Gly Thr Pro Gln Gly Ala Leu Glu Pro Cys Val
340 345 350

Gln Glu Ala Thr Ala Leu Leu Thr Cys Gly Pro Ala Arg Pro Trp Lys
355 360 365

Ser Val Ala Leu Glu Glu Glu Gln Glu Gly Pro Gly Thr Arg Leu Pro

370 375 380

Gly Asn Leu Ser Ser Glu Asp Val Leu Pro Ala Gly Cys Thr Glu Trp
385 390 395 400

Arg Val Gln Thr Leu Ala Tyr Leu Pro Gln Glu Asp Trp Ala Pro Thr
405 410 415

Ser Leu Thr Arg Pro Ala Pro Pro Asp Ser Glu Gly Ser Arg Ser Ser
420 425 430

Ser Ser Ser Ser Ser Ser Asn Asn Asn Asn Tyr Cys Ala Leu Gly Cys
435 440 445

Tyr Gly Gly Trp His Leu Ser Ala Leu Pro Gly Asn Thr Gln Ser Ser
450 455 460

Gly Pro Ile Pro Ala Leu Ala Cys Gly Leu Ser Cys Asp His Gln Gly
465 470 475 480

Leu Glu Thr Gln Gln Gly Val Ala Trp Val Leu Ala Gly His Cys Gln
485 490 495

Arg Pro Gly Leu His Glu Asp Leu Gln Gly Met Leu Leu Pro Ser Val
500 505 510

Leu Ser Lys Ala Arg Ser Trp Thr Phe
515 520

<210> 29
<211> 690
<212> DNA
<213> Homo sapiens

<400> 29
atgaccccgc agcttctcct ggcccttgtc ctctgggcca gctgcccgcc ctgcagtgga 60

aggaaagggc ccccagcagc tctgacactg ccccgggtgc aatgccgagc ctctcggtac 120

ccgatcgccg tggattgctc ctggaccctg ccgcctgctc caaactccac cagccccgtg 180

tccttcattg ccacgtacag gctcggcatg gctgcccggg gccacagctg gccctgcctg 240

cagcagacgc caacgtccac cagctgcacc atcacggatg tccagctgtt ctccatggct 300

ccctacgtgc tcaatgtcac cgccgtccac ccctggggct ccagcagcag cttcgtgcct 360

ttcataacag agcacatcat caagcccgac cctccagaag gcgtgcgcct aagccccctc 420

gctgagcgcc agctacaggt gcagtgggag cctcccgggt cctggccctt cccagagatc 480

ttctcactga agtactggat ccgttacaag cgtcagggag ctgcgcgctt ccaccgggtg 540

gggcccattg aagccacgtc cttcatcctc agggctgtgc ggccccgagc caggtactac 600

gtccaagtgg cggctcagga cctcacagac tacggggaac tgagtgactg gagtctcccc 660

gccactgcca caatgagcct gggcaagtag 690

<210> 30
<211> 210
<212> PRT
<213> Homo sapiens

<400> 30

Met Thr Pro Gln Leu Leu Leu Ala Leu Val Leu Trp Ala Ser Cys Pro
1 5 10 15

Pro Cys Ser Gly Arg Lys Gly Pro Pro Ala Ala Leu Thr Leu Pro Arg
20 25 30

Val Gln Cys Arg Ala Ser Arg Tyr Pro Ile Ala Val Asp Cys Ser Trp
35 40 45

Thr Leu Pro Pro Ala Pro Asn Ser Thr Ser Pro Val Ser Phe Ile Ala
50 55 60

Thr Tyr Arg Leu Gly Met Ala Ala Arg Gly His Ser Trp Pro Cys Leu
65 70 75 80

Gln Gln Thr Pro Thr Ser Thr Ser Cys Thr Ile Thr Asp Val Gln Leu
85 90 95

Phe Ser Met Ala Pro Tyr Val Leu Asn Val Thr Ala Val His Pro Trp
100 105 110

Gly Ser Ser Ser Ser Phe Val Pro Phe Ile Thr Glu His Ile Ile Lys
115 120 125

Pro Asp Pro Pro Glu Gly Val Arg Leu Ser Pro Leu Ala Glu Arg Gln
130 135 140

Leu Gln Val Gln Trp Glu Pro Pro Gly Ser Trp Pro Phe Pro Glu Ile
145 150 155 160

Phe Ser Leu Lys Tyr Trp Ile Arg Tyr Lys Arg Gln Gly Ala Ala Arg
165 170 175

Phe His Arg Val Gly Pro Ile Glu Ala Thr Ser Phe Ile Leu Arg Ala
180 185 190

Val Arg Pro Arg Ala Arg Tyr Tyr Val Gln Val Ala Ala Gln Asp Leu

195 200 205

Thr Asp
210

**Description**

FIELD OF THE INVENTION

The invention relates to a method of predicting a response of a prostate cancer subject to radiotherapy. Moreover, the invention relates to a diagnostic kit, to a use of the kit in a method of predicting a response of a prostate cancer subject to radiotherapy, to a use of a gene expression profile for each of one or more interleukin genes in radiotherapy prediction for a prostate cancer subject, and to a corresponding computer program product.

BACKGROUND OF THE INVENTION

Cancer is a class of diseases in which a group of cells displays uncontrolled growth, invasion and sometimes metastasis. These three malignant properties of cancers differentiate them from benign tumours, which are self-limited and do not invade or metastasize. Prostate Cancer (PCa) is the second most commonly-occurring non-skin malignancy in men, with an estimated 1.3 million new cases diagnosed and 360,000 deaths world-wide in 2018 (see Bray F. et al., "Global cancer statistics 2018: GLOBOCAN estimates of incidence and mortality worldwide for 36 cancers in 185 countries", CA Cancer J Clin, Vol. 68, No. 6, pages 394-424, 2018). In the US, about 90% of the new cases concern localized cancer, meaning that metastases have not yet been formed (see ACS (American Cancer Society), "Cancer Facts & Figures 2010", 2010).

For the treatment of primary localized prostate cancer, several radical therapies are available, of which surgery (radical prostatectomy, RP) and radiation therapy (RT) are most commonly used. RT is administered via an external beam or via the implantation of radioactive seeds into the prostate (brachytherapy) or a combination of both. It is especially preferable for patients who are not eligible for surgery or have been diagnosed with a tumour in an advanced localized or regional stage. Radical RT is provided to up to 50% of patients diagnosed with localized prostate cancer in the US (see ACS, 2010, ibid).

After treatment, prostate cancer antigen (PSA) levels in the blood are measured for disease monitoring. An increase of the blood PSA level provides a biochemical surrogate measure for cancer recurrence or progression. However, the variation in reported biochemical progression-free survival (bPFS) is large (see Grimm P. et al., "Comparative analysis of prostate-specific antigen free survival outcomes for patients with low, intermediate and high risk prostate cancer treatment by radical therapy. Results from the Prostate Cancer Results Study Group", BJU Int, Suppl. 1, pages 22-29, 2012). For many patients, the bPFS at 5 or even 10 years after radical RT may lie above 90%. Unfortunately, for the group of patients at medium and especially higher risk of recurrence, the bPFS can drop to around 40% at 5 years, depending on the type of RT used (see Grimm P. et al., 2012, ibid).

A large number of the patients with primary localized prostate cancer that are not treated with RT will undergo RP (see ACS, 2010, ibid). After RP, an average of 60% of patients in the highest risk group experience biochemical recurrence after 5 and 10 years (see Grimm P. et al., 2012, ibid). In case of biochemical progression after RP, one of the main challenges is the uncertainty whether this is due to recurring localized disease, one or more metastases or even an indolent disease that will not lead to clinical disease progression (see Dal Pra A. et al., "Contemporary role of postoperative radiotherapy for prostate cancer", Transl Androl Urol, Vo. 7, No. 3, pages 399-413, 2018, and Herrera F.G. and Berthold D.R., "Radiation therapy after radical prostatectomy: Implications for clinicians", Front Oncol, Vol. 6, No. 117, 2016). RT to eradicate remaining cancer cells in the prostate bed is one of the main treatment options to salvage survival after a PSA increase following RP. The effectiveness of salvage radiotherapy (SRT) results in 5-year bPFS for 18% to 90% of patients, depending on multiple factors (see Herrera F.G. and Berthold D.R., 2016, ibid, and Pisansky T.M. et al., "Salvage radiation therapy dose response for biochemical failure of prostate cancer after prostatectomy - A multi-institutional observational study", Int J Radiat Oncol Biol Phys, Vol. 96, No. 5, pages 1046-1053, 2016).

It is clear that for certain patient groups, radical or salvage RT is not effective. Their situation is even worsened by the serious side effects that RT can cause, such as bowel inflammation and dysfunction, urinary incontinence and erectile dysfunction (see Resnick M.J. et al., "Long-term functional outcomes after treatment for localized prostate cancer", N Engl J Med, Vol. 368, No. 5, pages 436-445, 2013, and Hegarty S.E. et al., "Radiation therapy after radical prostatectomy for prostate cancer: Evaluation of complications and influence of radiation timing on outcomes in a large, population-based cohort", PLoS One, Vol. 10, No. 2, 2015). In addition, the median cost of one course of RT based on Medicare reimbursement is $ 18,000, with a wide variation up to about $ 40,000 (see Paravati A.J. et al., "Variation in the cost of radiation therapy among medicare patients with cancer", J Oncol Pract, Vol. 11, No. 5, pages 403-409, 2015). These figures do not include the considerable longitudinal costs of follow-up care after radical and salvage RT.

An improved prediction of effectiveness of RT for each patient, be it in the radical or the salvage setting, would improve therapy selection and potentially survival. This can be achieved by 1) optimizing RT for those patients where RT is predicted to be effective (e.g., by dose escalation or a different starting time) and 2) guiding patients where RT is predicted not to be effective to an alternative, potentially more effective form of treatment. Further, this would reduce suffering for those patients who would be spared ineffective therapy and would reduce costs spent on ineffective therapies.

Numerous investigations have been conducted into measures for response prediction of radical RT (see Hall W.A. et al., "Biomarkers of outcome in patients with localized prostate cancer treated with radiotherapy", Semin Radiat Oncol, Vol. 27, pages 11-20, 2016, and Raymond E. et al., "An appraisal of analytical tools used in predicting clinical outcomes following radiation therapy treatment of men with prostate cancer: A systematic review", Radiat Oncol, Vol. 12, No. 1, page 56, 2017) and SRT (see Herrera F.G. and Berthold D.R., 2016, ibid). Many of these measures depend on the concentration of the blood-based biomarker PSA. Metrics investigated for prediction of response before start of RT (radical as well as salvage) include the absolute value of the PSA concentration, its absolute value relative to the prostate volume, the absolute increase over a certain time and the doubling time. Other frequently considered factors are the Gleason score and the clinical tumour stage. For the SRT setting, additional factors are relevant, e.g., surgical margin status, time to recurrence after RP, pre-/peri-surgical PSA values and clinico-pathological parameters.

Although these clinical variables provide limited improvements in patient stratification in various risk groups, there is a need for better predictive tools.

A wide range of biomarker candidates in tissue and bodily fluids has been investigated, but validation is often limited and generally demonstrates prognostic information and not a predictive (therapy-specific) value (see Hall W.A. et al., 2016, ibid). A small number of gene expression panels is currently being validated by commercial organizations. One or a few of these may show predictive value for RT in future (see Dal Pra A. et al., 2018, ibid).

In conclusion, a strong need for better prediction of response to RT remains, for primary prostate cancer as well as for the post-surgery setting.

## SUMMARY OF THE INVENTION

It is an objective of the invention to provide a method of predicting a response of a prostate cancer subject to radiotherapy, which allows to make better treatment decisions. It is a further objective of the invention to provide a diagnostic kit, a use of the kit in a method of predicting a response of a prostate cancer subject to radiotherapy, a use of a gene expression profile for each of one or more interleukin genes in radiotherapy prediction for a prostate cancer subject, and a corresponding computer program product.

In a first aspect of the present invention, a method of predicting a response of a prostate cancer subject to radiotherapy is presented, comprising:

- determining or receiving the result of a determination of a gene expression profile for each of one or more, for example, 1, 2, 3, 4, 5 or all, interleukin genes selected from the group consisting of: IL17RE, IL1B, IL3, IL7R, IL9R, and EBI3, said gene expression profile(s) being determined in a biological sample obtained from the subject,
- determining the prediction of the radiotherapy response based on the gene expression profile(s) for the one or more interleukin genes, and
- optionally, providing the prediction or a therapy recommendation based on the prediction to a medical caregiver or the subject.

In recent years, the importance of the immune system in cancer inhibition as well as in cancer initiation, promotion and metastasis has become very evident (see Mantovani A. et al., "Cancer-related inflammation", Nature, Vol. 454, No. 7203, pages 436-444, 2008, and Giraldo N.A. et al., "The clinical role of the TME in solid cancer", Br J Cancer, Vol. 120, No. 1, pages 45-53, 2019). The immune cells and the molecules they secrete form a crucial part of the tumour microenvironment and most immune cells can infiltrate the tumour tissue. The immune system and the tumour affect and shape one another. Thus, anti-tumour immunity can prevent tumour formation while an inflammatory tumour environment may promote cancer initiation and proliferation. At the same time, tumour cells that may have originated in an immune system-independent manner will shape the immune microenvironment by recruiting immune cells and can have a pro-inflammatory effect while also suppressing anti-cancer immunity.

Some of the immune cells in the tumour microenvironment will have either a general tumour-promoting or a general tumour-inhibiting effect, while other immune cells exhibit plasticity and show both tumour-promoting and tumour-inhibiting potential. Thus, the overall immune microenvironment of the tumour is a mixture of the various immune cells present, the cytokines they produce and their interactions with tumour cells and with other cells in the tumour microenvironment (see Giraldo N.A. et al., 2019, ibid).

The principles described above with regard to the role of the immune system in cancer in general also apply to prostate cancer. Chronic inflammation has been linked to the formation of benign as well as malignant prostate tissue (see Hall W.A. et al., 2016, ibid) and most prostate cancer tissue samples show immune cell infiltrates. The presence of specific immune cells with a pro-tumour effect has been correlated with worse prognosis, while tumours in which natural killer cells were more activated showed better response to therapy and longer recurrence-free periods (see Shiao S.L. et al., "Regulation of prostate cancer progression by tumor microenvironment", Cancer Lett, Vol. 380, No. 1, pages 340-348, 2016).

While a therapy will be influenced by the immune components of the tumour microenvironment, RT itself extensively affects the make-up of these components (see Barker H.E. et al., "The tumor microenvironment after radiotherapy: Mechanisms of resistance or recurrence", Nat Rev Cancer, Vol. 15, No. 7, pages 409-425, 2015). Because suppressive cell types are comparably radiation-insensitive, their relative numbers will increase. Counteractively, the inflicted radiation damage activates cell survival pathways and stimulates the immune system, triggering inflammatory responses and immune cell recruitment. Whether the net effect will be tumour-promoting or tumour-suppressing is as yet uncertain, but its potential for enhancement of cancer immunotherapies is being investigated.

The present invention is based on the idea that, since the status of the immune system and of the immune microenvironment have an impact on therapy effectiveness, the ability to identify markers predictive for this effect might help to be better able to predict overall RT response.

Interleukins are one of the main groups of cytokines. They form a large family of over 50 molecules that play a central role in the regulation of the immune system (see Brocker C. et al., "Evolutionary divergence and functions of the human interleukin (IL) gene family", Hum Genomics, Vol. 5, No. 1, pages 30-55, 2010). Based on their sequence, interleukins can be clustered in 4 major groups, but overall their sequence similarity is relatively weak. The main function of these proteins is to modulate growth, differentiation and activation during an immune response. Multiple family members are involved in the activation or suppression of T cells, which in their turn may play a role in, for example, clearing of tumour cells or the modulation of inflammatory responses after RT.

Interleukins exert their function by enabling communication between cells. They are secreted by immune cells and reach their target cells via interstitial fluid and the blood circulation. The target cells express interleukin receptor molecules on their surface to which the interleukins can bind, thereby activating or inhibiting a signalling cascade inside the cell. The signalling cascade finally influences the expression of proteins resulting in immune cell growth, differentiation and activation.

Which function interleukins exactly perform depends on the secreting cells, the target cells and the phase of the immune response. One interleukin can bind to multiple different receptors. Moreover, an interleukin can have both pro- and anti-inflammatory effects. This highly complicates the determination of the precise functions of each interleukin. Therefore, it is extremely difficult to conclude based on literature which members of the interleukin family or their receptors might be specifically predictive for the response of prostate cancer patients to radical RT or SRT.

Several investigations have been performed as to the change in levels of one or more interleukins during RT for prostate cancer, or even therapy targeting an interleukin. Very recently, a report was made on the investigation of a number of interleukins in serum for the prediction of RT response in prostate cancer (see Hall W.A. et al., "The influence of the pretreatment host immune inflammatory state and response to radiation therapy in high-risk adenocarcinoma of the prostate: A validation study from NRG Oncology/RTOG 0521"), Vol. 102, No. 3, pages S13-S14, 2018). A link with disease free survival was found for the level of IL10 in serum. A few other interleukins were found to be linked not to survival but to side effects of radiation. This highlights the need for a complete overview of multiple IL components and their activity.

The identified interleukin genes IL17RE, IL1B, IL3, IL7R, IL9R, and EBI3 were identified as follows: A group of 538 prostate cancer patients were treated with RP and the prostate cancer tissue was stored. A number of these patients experienced biochemical recurrence and was treated with SRT. For 151 of these patients, the RNA expressed in the originally stored prostate cancer tissue was analysed using RNA sequencing. The mRNA expression of interleukins and their receptors was compared for the 26 out of 151 patients that died due to prostate cancer, versus the 125 out of 151 patients who survived. For the six molecules IL17RE, IL1B, IL3, IL7R, IL9R, and EBI3, the expression level was significantly different for the survivors, suggesting that they have value in the prediction of survival after SRT. Several of these six molecules were differentially expressed in other data sets as well, as described in more detail further below.

The term "IL17RE" refers to the human Interleukin 17 Receptor E gene (Ensembl: ENSG00000163701), for example, to the sequence as defined in NCBI Reference Sequence NM_153480.2 or in NCBI Reference Sequence NM_001193380.2, specifically, to the nucleotide sequence as set forth in SEQ ID NO:1 or in SEQ ID NO:2, which correspond to the sequences of the above indicated NCBI Reference Sequences of the IL17RE transcript, and also relates to the corresponding amino acid sequence for example as set forth in SEQ ID NO:3 or in SEQ ID NO:4, which correspond to the protein sequences defined in NCBI Protein Accession Reference Sequence NP_705613.1 and in NCBI Protein Accession Reference Sequence NP_001180309.1 encoding the IL17RE polypeptide.

The term "IL17RE" also comprises nucleotide sequences showing a high degree of homology to IL17RE, e.g., nucleic acid sequences being at least 75%, 80%, 85%, 90%, 91%, 92%, 93%, 94%, 95%, 96%, 97%, 98% or 99% identical to the sequence as set forth in SEQ ID NO:1 or in SEQ ID NO:2 or amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 1 or in SEQ ID NO:2.

Alternatively, the term "IL117RE" refers to artificial variants of a naturally occurring IL17RE. For example, consensus or core sequences over several isoform sequences can be computationally mapped and defined. Examples of such computationally mapped potential isoform sequences are set forth in SEQ ID NO:5, in SEQ ID NO:6, in SEQ ID NO:7, in SEQ ID NO:8 and in SEQ ID NO:9. A sequence comprising this sequence, or a sequence having at least 90%, 91%, 92%, 93%, 94%, 95%, 96%, 97%, 98% or 99% sequence identity thereto also means an IL17RE in the sense of the present disclosure.

The term "IL1B" refers to the human Interleukin 1 Beta gene (Ensembl: ENSG00000125538), for example, to the sequence as defined in NCBI Reference Sequence NM_000576.3, specifically, to the nucleotide sequence as set forth in SEQ ID NO:10, which corresponds to the sequence of the above indicated NCBI Reference Sequence of the IL1B transcript, and also relates to the corresponding amino acid sequence for example as set forth in SEQ ID NO: 11, which corresponds to the protein sequence defined in NCBI Protein Accession Reference Sequence NP_000567.1 encoding the IL1B polypeptide.

The term "IL1B" also comprises nucleotide sequences showing a high degree of homology to IL1B, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 10 or amino acid sequences being at least 75%, 80%, 85%, 90%, 91%, 92%, 93%, 94%, 95%, 96%, 97%, 98% or 99% identical to the sequence as set forth in SEQ ID NO:11 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:11 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 10.

Alternatively, the term "IL1B" refers to artificial variants of a naturally occurring IL1B. For example, consensus or core sequences over several isoform sequences can be computationally mapped and defined. Examples of such computationally mapped potential isoform sequences are set forth in SEQ ID NO:12, in SEQ ID NO:13 and in SEQ ID NO:14. A sequence comprising this sequence, or a sequence having at least 90%, 91%, 92%, 93%, 94%, 95%, 96%, 97%, 98% or 99% sequence identity thereto also means an IL1B in the sense of the present disclosure.

The term "IL3" refers to the human Interleukin 3 gene (Ensembl: ENSG00000164399), for example, to the sequence as defined in NCBI Reference Sequence NM_000588.4, specifically, to the nucleotide sequence as set forth in SEQ ID NO: 15, which corresponds to the sequence of the above indicated NCBI Reference Sequence of the IL3 transcript, and also relates to the corresponding amino acid sequence for example as set forth in SEQ ID NO:16, which corresponds to the protein sequence defined in NCBI Protein Accession Reference Sequence NP_000579.2 encoding the IL3 polypeptide.

The term "IL3" also comprises nucleotide sequences showing a high degree of homology to IL3, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 15 or amino acid sequences being at least 75%, 80%, 85%, 90%, 91%, 92%, 93%, 94%, 95%, 96%, 97%, 98% or 99% identical to the sequence as set forth in SEQ ID NO:16 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:16 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 15.

The term "IL7R" refers to the human Interleukin 7 Receptor gene (Ensembl: ENSG00000168685), for example, to the sequence as defined in NCBI Reference Sequence NM_002185.5, specifically, to the nucleotide sequence as set forth in SEQ ID NO:17, which corresponds to the sequence of the above indicated NCBI Reference Sequence of the IL7R transcript, and also relates to the corresponding amino acid sequence for example as set forth in SEQ ID NO:18, which corresponds to the protein sequence defined in NCBI Protein Accession Reference Sequence NP_002176.2 encoding the IL7R polypeptide.

The term "IL7R" also comprises nucleotide sequences showing a high degree of homology to IL7R, e.g., nucleic acid sequences being at least 75%, 80%, 85%, 90%, 91%, 92%, 93%, 94%, 95%, 96%, 97%, 98% or 99% identical to the sequence as set forth in SEQ ID NO:17 or amino acid sequences being at least 75%, 80%, 85%, 90%, 91%, 92%, 93%, 94%, 95%, 96%, 97%, 98% or 99% identical to the sequence as set forth in SEQ ID NO:18 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 17.

Alternatively, the term "IL7R" refers to artificial variants of a naturally occurring IL7R. For example, consensus or core sequences over several isoform sequences can be computationally mapped and defined. Examples of such computationally mapped potential isoform sequences are set forth in SEQ ID NO:19, in SEQ ID NO:20, in SEQ ID NO:21, in SEQ ID NO:22, in SEQ ID NO:23 and in SEQ ID NO:24. A sequence comprising this sequence, or a sequence having at least 90%, 91%, 92%, 93%, 94%, 95%, 96%, 97%, 98% or 99% sequence identity thereto also means an IL7R in the sense of the present disclosure.

The term "IL9R" refers to the human Interleukin 9 Receptor gene (ENSG00000124334), for example, to the sequence as defined in NCBI Reference Sequence NM_176786.2 or in NCBI Reference Sequence NM_002186.3, specifically, to the nucleotide sequence as set forth in SEQ ID NO:25 or in SEQ ID NO:26, which correspond to the sequences of the above indicated NCBI Reference Sequences of the IL9R transcript, and also relates to the corresponding amino acid sequence for example as set forth in SEQ ID NO:27 or in SEQ ID NO:28, which correspond to the protein sequences defined in NCBI Protein Accession Reference Sequence NP_789743.2 and in NCBI Protein Accession Reference Sequence NP_002177.2 encoding the IL9R polypeptide.

The term "IL9R" also comprises nucleotide sequences showing a high degree of homology to IL9R, e.g., nucleic acid sequences being at least 75%, 80%, 85%, 90%, 91%, 92%, 93%, 94%, 95%, 96%, 97%, 98% or 99% identical to the sequence as set forth in SEQ ID NO:25 or in SEQ ID NO:26 or amino acid sequences being at least 75%, 80%, 85%, 90%, 91%, 92%, 93%, 94%, 95%, 96%, 97%, 98% or 99% identical to the sequence as set forth in SEQ ID NO:27 or in SEQ ID NO:28 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:27 or in SEQ ID NO:28 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:25 or in SEQ ID NO:26.

The term "EBI3" refers to the human Epstein-Barr Virus Induced 3 gene (Ensembl: ENSG00000105246), for example, to the sequence as defined in NCBI Reference Sequence NM_005755.3, specifically, to the nucleotide sequence as set forth in SEQ ID NO:29, which corresponds to the sequence of the above indicated NCBI Reference Sequence of the EBI3 transcript, and also relates to the corresponding amino acid sequence for example as set forth in SEQ ID NO:30, which corresponds to the protein sequence defined in NCBI Protein Accession Reference Sequence NP_005746.2 encoding the EBI3 polypeptide.

The term "EBI3" also comprises nucleotide sequences showing a high degree of homology to EBI3, e.g., nucleic acid sequences being at least 75%, 80%, 85%, 90%, 91%, 92%, 93%, 94%, 95%, 96%, 97%, 98% or 99% identical to the sequence as set forth in SEQ ID NO:29 or amino acid sequences being at least 75%, 80%, 85%, 90%, 91%, 92%, 93%, 94%, 95%, 96%, 97%, 98% or 99% identical to the sequence as set forth in SEQ ID NO:30 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:30 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:29.

The term "biological sample" or "sample obtained from a subject" refers to any biological material obtained via suitable methods known to the person skilled in the art from a subject, e.g., a prostate cancer patient. The biological sample used may be collected in a clinically acceptable manner, e.g., in a way that nucleic acids (in particular RNA) or proteins are preserved.

The biological sample(s) may include body tissue and/or a fluid, such as, but not limited to, blood, sweat, saliva, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the prostate of a male subject. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing prostate secreted exosomes, or cell lines or cancer cell line.

In one particular realization, biopsy or resections samples may be obtained and/or used. Such samples may include cells or cell lysates.

It is also conceivable that the content of a biological sample is submitted to an enrichment step. For instance, a sample may be contacted with ligands specific for the cell membrane or organelles of certain cell types, e.g., prostate cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

The term "prostate cancer" refers to a cancer of the prostate gland in the male reproductive system, which occurs when cells of the prostate mutate and begin to multiply out of control. Typically, prostate cancer is linked to an elevated level of prostate-specific antigen (PSA). In one embodiment of the present invention the term "prostate cancer" relates to a cancer showing PSA levels above 3.0. In another embodiment the term relates to cancer showing PSA levels above 2.0. The term "PSA level" refers to the concentration of PSA in the blood in ng/ml.

The term "non-progressive prostate cancer state" means that a sample of an individual does not show parameter values indicating "biochemical recurrence" and/or "clinical recurrence" and/or "metastases" and/or "castration-resistant disease" and/or "prostate cancer or disease specific death".

The term "progressive prostate cancer state" means that a sample of an individual shows parameter values indicating "biochemical recurrence" and/or "clinical recurrence" and/or "metastases" and/or "castration-resistant disease" and/or "prostate cancer or disease specific death".

The term "biochemical recurrence" generally refers to recurrent biological values of increased PSA indicating the presence of prostate cancer cells in a sample. However, it is also possible to use other markers that can be used in the detection of the presence or that rise suspicion of such presence.

The term "clinical recurrence" refers to the presence of clinical signs indicating the presence of tumour cells as measured, for example using in vivo imaging.

The term "metastases" refers to the presence of metastatic disease in organs other than the prostate.

The term "castration-resistant disease" refers to the presence of hormone-insensitive prostate cancer; i.e., a cancer in the prostate that does not any longer respond to androgen deprivation therapy (ADT).

The term "prostate cancer specific death or disease specific death" refers to death of a patient from his prostate cancer.

It is preferred that the one or more interleukin genes comprise three or more, preferably, all of the interleukin genes.

It is preferred that the determining of the prediction of the radiotherapy response comprises combining the gene expression profiles for two or more, for example, 2, 3, 4, 5 or all, of the interleukin genes with a regression function that had been derived from a population of prostate cancer subjects.

Regression analysis helps one understand how the typical value of the dependent variable (or "criterion variable") changes when any one of the independent variables is varied, while the other independent variables are held fixed. This relationship between the dependent variable and the independent variables is captured in the regression function, which can be used to predict the dependent variable given the values of the independent variables. The dependent variable can be, for example, a binary variable, such as biochemical relapse within 5 years after radiotherapy. In this case, the regression is a logistic regression that is based on a logit function of the independent variables, which, here, comprise or consist of the gene expression profiles for two or more of the interleukin genes. By means of the regression function, an improved prediction of e.g. the 5-year risk of biochemical recurrence after radiotherapy may be possible.

In one particular realization, the prediction of the radiotherapy response is determined as follows:

$$c + (w_1 \cdot IL17RE) + (w_2 \cdot IL1B) + (w_3 \cdot IL3) + (w_4 \cdot IL7R) + (w_5 \cdot IL9R) + (w_6 \cdot EBI3) \quad (1)$$

where $w_1$ to $w_6$ are weights, c is a constant, and IL17RE, IL1B, IL3, IL7R, IL9R, and EBI3 are the expression levels of the interleukin genes.

In one example, $w_1$ may be about 0.5 to 1.5, such as 9.94141, $w_2$ may be about -2.0 to -1.0, such as -1.42739, $w_3$ may be about 1.0 to 2.0, such as 1.26008, $w_4$ may be about -2.5 to -1.5, such as -1.91264, $w_5$ may be about 0.0 to 1.0, such as 0.50106, $w_6$ may be about 4.0 to 6.0, such as 5.10369, and c may be about -6.0 to -4.0, such as -5.174.

The prediction of the radiotherapy response may also be classified or categorized into one of at least two risk groups, based on the value of the prediction of the radiotherapy response. For example, there may be two risk groups, or three risk groups, or four risk groups, or more than four predefined risk groups. Each risk group covers a respective range of (non-overlapping) values of the prediction of the radiotherapy response. For example, a risk group may indicate a probability of occurrence of a specific clinical event from 0 to <0.1 or from 0.1 to <0.25 or from 0.25 to <0.5 or from 0.5 to 1.0 or the like.

It is further preferred that the determining of the prediction of the radiotherapy response is further based on one or more clinical parameters obtained from the subject.

As mentioned above, various measures based on clinical parameters have been investigated. By further basing the prediction of the radiotherapy response on such clinical parameter(s), it can be possible to further improve the prediction.

It is preferred that the one or more clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

It is further preferred that the determining of the prediction of the radiotherapy response comprises combining the gene expression profile(s) for the one or more interleukin genes and the one or more clinical parameters obtained from the subject with a regression function that had been derived from a population of prostate cancer subjects.

In one particular realization, the prediction of the radiotherapy response is determined as follows:

$$c + (w_1 \cdot IL17RE) + (w_2 \cdot IL1B) + (w_3 \cdot IL3) + (w_4 \cdot IL7R) + (w_5 \cdot IL9R) + (w_6 \cdot EBI3) + (w_7 \cdot pGGG) \quad (2)$$

where $w_1$ to $w_7$ are weights, c is a constant, IL17RE, IL1B, IL3, IL7R, IL9R and EBI3 are the expression levels of the interleukin genes, and pGGG is the pathological Gleason grade group.

In one example, $w_1$ may be about 1.5 to 2.5, such as 1.88159, $w_2$ may be about -0.5 to 0.5, such as -0.041318, $w_3$ may be about 0.0 to 1.0, such as 0.59493, $w_4$ may be about -5.0 to -4.0, such as -4.75981, $w_5$ may be about -1.5 to -0.5, such as -0.84648, $w_6$ may be about 3.5 to 5.5, such as 4.56796, $w_7$ may be about 2.0 to 3.0, such as 2.37674, and c may be about -8.5 to -6.5, such as -7.60381.

It is preferred that the biological sample is obtained from the subject before the start of the radiotherapy. The gene expression profile(s) may be determined in the form of mRNA or protein in tissue of prostate cancer. Alternatively, if the interleukins are present in a soluble form, the gene expression profile(s) may be determined in blood.

It is further preferred that the radiotherapy is radical radiotherapy or salvage radiotherapy.

It is preferred that the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy. The degree to which the prediction is negative may determine the degree to which the recommended therapy deviates from the standard form of radiotherapy.

In a further aspect of the present invention, an apparatus for predicting a response of a prostate cancer subject to radiotherapy is presented, comprising:

- an input adapted to receive data indicative of a gene expression profile for each of one or more, for example, 1, 2, 3, 4, 5 or all, interleukin genes selected from the group consisting of: IL17RE, IL1B, IL3, IL7R, IL9R, and EBI3, said gene expression profile(s) being determined in a biological sample obtained from the subject,
- a processor adapted to determine the prediction of the radiotherapy response based on the gene expression profile(s) for the one or more interleukin genes, and
- optionally, a providing unit adapted to provide the prediction or a therapy recommendation based on the prediction to a medical caregiver or the subject.

In a further aspect of the present invention, a computer program product is presented comprising instructions which, when the program is executed by a computer, cause the computer to carry out a method comprising:

- receiving data indicative of a gene expression profile for each of one or more, for example, 1, 2, 3, 4, 5 or all, interleukin genes selected from the group consisting of: IL17RE, IL1B, IL3, IL7R, IL9R, and EBI3, said gene expression profile(s) being determined in a biological sample obtained from a prostate cancer subject,
- determining the prediction of the radiotherapy response based on the gene expression profile(s) for the one or more interleukin genes, and
- optionally, providing the prediction or a therapy recommendation based on the prediction to a medical caregiver or the subject.

In a further aspect of the present invention, a diagnostic kit is presented, comprising:

- at least one primer and/or probe for determining the gene expression profile for each of one or more, for example, 1, 2, 3, 4, 5 or all, interleukin genes selected from the group consisting of: IL17RE, IL1B, IL3, IL7R, IL9R, and EBI3, in a biological sample obtained from the subject, and
- optionally, an apparatus as defined in claim 10 or a computer program product as defined in claim 11.

In a further aspect of the present invention, a use of the kit as defined in claim 12 is presented.

It is preferred that the use as defined in claim 13 is in a method of predicting a response of a prostate cancer subject to radiotherapy.

In a further aspect of the present invention, a method is presented, comprising:

- receiving a biological sample obtained from a prostate cancer subject,
- using the kit as defined in claim 12 to determine a gene expression profile for each of one or more, for example, 1, 2, 3, 4, 5 or all, interleukin genes selected from the group consisting of: IL17RE, IL1B, IL3, IL7R, IL9R, and EBI3, in the biological sample obtained from the subject.

In a further aspect of the present invention, a use of a gene expression profile for each of one or more, for example, 1, 2, 3, 4, 5 or all, interleukin genes selected from the group consisting of: IL17RE, IL1B, IL3, IL7R, IL9R, and EBI3, in a method of predicting a response of a prostate cancer subject to radiotherapy is presented, comprising

- determining the prediction of the radiotherapy response based on the gene expression profile(s) for the one or more interleukin genes, and
- optionally, providing the prediction or a therapy recommendation based on the prediction to a medical caregiver or the subject.

It shall be understood that the method of claim 1, the apparatus of claim 10, the computer program product of claim 11, the diagnostic kit of claim 12, the use of the diagnostic kit of claim 13, the method of claim 15, and the use of a gene expression profile(s) of claim 16 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily a flowchart of an embodiment of a method of predicting a response of a prostate cancer subject to radiotherapy.

Fig. 2 shows a ROC curve analysis of three predictive models.

Fig. 3 shows a Kaplan-Meier curve analysis of the Interleukin model (IL_model). The clinical endpoint that was tested was the time to metastases (TTM) after the start of salvage radiation therapy (SRT) due to post-surgical disease recurrence.

Fig. 4 shows a Kaplan-Meier curve analysis of the Interleukin model & pGGG combination model (IL&pGGG_model). The clinical endpoint that was tested was the time to metastases (TTM) after the start of salvage radiation therapy (SRT) due to post-surgical disease recurrence.

Fig. 5 shows a Kaplan-Meier curve analysis of the Interleukin model (IL_model). The clinical endpoint that was tested was the time to prostate cancer specific death (PCa Death) after the start of the salvage radiation therapy (SRT) due to post-surgical disease recurrence.

Fig. 6 shows a Kaplan-Meier curve of the Interleukin model & pGGG combination model (IL&pGGG_model). The clinical endpoint that was tested was the time to prostate cancer specific death (PCa Death) after the start of salvage radiation therapy (SRT) due to post-surgical disease recurrence.

DETAILED DESCRIPTION OF EMBODIMENTS

**Overview Of Radiotherapy Response Prediction**

Fig. 1 shows schematically and exemplarily a flowchart of an embodiment of a method of predicting a response of a prostate cancer subject to radiotherapy. The method begins at step S100.

At step S102, a biological sample is obtained from each of a first set of patients (subjects) diagnosed with prostate cancer. Preferably, monitoring prostate cancer has been performed for these prostate cancer patients over a period of time, such as at least one year, or at least two years, or about five years, after obtaining the biological sample.

At step S104, a gene expression profile for each of two or more, for example, 2, 3, 4, 5 or all, interleukin genes selected from the group consisting of: IL17RE, IL1B, IL3, IL7R, IL9R, and EBI3, is obtained for each of the biological samples obtained from the first set of patients, e.g., by performing RT-qPCR (real-time quantitative PCR) on RNA extracted from each biological sample. The exemplary gene expression profiles include an expression level (e.g., value) for each of the two or more interleukin genes.

At step S106, a regression function for assigning a prediction of the radiotherapy response is determined based on the gene expression profiles for the two or more interleukin genes, IL17RE, IL1B, IL3, IL7R, IL9R, and/or EBI3, obtained for at least some of the biological samples obtained for the first set of patients and respective results obtained from the monitoring. In one particular realization, the regression function is determined as specified in Eq. (1) above.

At step S108, a biological sample is obtained from a patient (subject or individual). The patient can be a new patient or one of the first set.

At step S110, a gene expression profile is obtained for each of the two or more, for example, 2, 3, 4, 5 or all, interleukin genes, e.g., by performing PCR on the biological sample.

At step S112, a prediction of the radiotherapy response based on the gene expression profiles for the two or more interleukin genes is determined for the patient using the regression function. This will be described in more detail later in the description.

At S114, a therapy recommendation may be provided, e.g., to the patient or his or her guardian, to a doctor, or to another healthcare worker, based on the prediction. To this end, the prediction may be categorized into one of a predefined set of risk groups, based on the value of the prediction. In one particular realization, the prediction of the radiotherapy response may be negative or positive for the effectiveness of the radiotherapy. If the prediction is negative, the recommended therapy may comprise one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

The method ends at S116.

In one embodiment, the gene expression profiles at steps S104 and S110 are determined by detecting mRNA expression using two or more primers and/or probes and/or two or more sets thereof.

In one embodiment, steps S104 and S110 further comprise obtaining one or more clinical parameters from the first set of patients and the patient, respectively. The one or more clinical parameters may comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score. The regression function for assigning the prediction of the radiotherapy response that is determined in step S106 is then further based on the one or more clinical parameters obtained from at least some of the first set of patients. In step S112, the prediction of the radiotherapy response is then further based on the one or more clinical parameters, e.g., the pathological Gleason grade group (pGGG), obtained from the patient and is determined for the patient using the regression function.

The immune system interacts in a strong manner with prostate cancer, both on a systemic level and in the tumour microenvironment. Interleukins play a central role in the regulation of immune activity. Interleukins and their receptors may therefore provide information on the effectiveness of RT. However, which members of the interleukin family may have predictive value in this application is extremely difficult to deduce from existing literature due to the many factors that influence the exact function of interleukins.

We investigated the extent to which the expression of interleukins and their receptors in prostate cancer tissue correlates with the recurrence of disease after radical RT or SRT.

We have identified six members of the family of interleukins and interleukin receptors for which the degree of expression in prostate cancer tissue significantly correlates with mortality after SRT, in a cohort of 151 prostate cancer patients. For two of these interleukins, we found also a significant correlation of expression with disease recurrence in an independent cohort of 248 patients treated with radical RT for primary localized prostate cancer. One of these two interleukin receptors showed in addition a significant correlation with the occurrence of metastases after SRT in the first cohort of 151 patients.

Based on the significant correlation with outcome after RT, we expect that the identified molecules will provide predictive value with regard to the effectiveness of radical RT and/or SRT.

TABLE 1: Univariate Cox regression analysis of two independent prostate cancer patient cohorts. The number of patients are indicated per cohort. The tested endpoint in the two cohorts is post-treatment progression free survival for men undergoing salvage radiation (SRT) after post-surgical disease recurrence (cohort #1) vs. men stratified to radical radiation (RRT) as the primary, localized therapy (cohort #2). The number of events and percentage per endpoint is indicated in parenthesis. The table indicates for each tested interleukin gene the association in terms of risk (Hazard Ratio) and significance (p-value) to the tested endpoints.

| Data Set | #1 (Prostate RNAseq) | | | | #2 (GSE116918) | | | |
|---|---|---|---|---|---|---|---|---|
| # Patients | 151 | | | | 248 | | | |
| Outcome | Post-Salvage-Radiation Outcome | | | | Post-Primary-Radiation Outcome | | | |
| Endpoint (# events / # patients; % events) | Metastases (#65/#151; 43.0%) | | Prostate Cancer Mortality (#26/#151; 17.2%) | | Biochemical Relapse (#56/#248; 22.6%) | | Metastases (#22/#248; 8.9%) | |
| Interleukin | p-value | HR | p-value | HR | p-value | HR | p-value | HR |
| IL17RE | 0.003 | 1.84 | 0.0009 | 2.26 | 0.029 | 2.26 | 0.049 | 3.14 |
| IL1B | 0.740 | 1.07 | 0.0081 | 0.21 | 0.533 | 1.08 | 0.070 | 1.31 |
| IL3 | 0.100 | 1.35 | 0.018 | 1.56 | 0.843 | 0.92 | 0.832 | 1.15 |
| IL7R | 0.550 | 0.78 | 0.023 | 0.12 | 0.256 | 1.15 | 0.350 | 1.20 |
| IL9R | 0.100 | 1.42 | 0.025 | 1.71 | 0.086 | 1.55 | 0.043 | 2.03 |
| EBI3 | 0.250 | 2.49 | 0.019 | 6.76 | 0.020 | 2.30 | 0.119 | 2.42 |

Compared to Hall and colleagues (see Hall W.A. et al., 2018, ibid), the number of interleukins we tested was larger, encompassing all known interleukins and their receptors. For example, the molecules IL17RE, EBI3, IL7R, IL9R and IL3, which we found to have predictive value, were not analysed by Hall W.A. et al., 2018. Another difference is that they analysed proteins in blood serum, while we analysed mRNA in tissue. We found a predictive value for IL1B but not for IL10, contrary to the findings of Hall W.A. et al., 2018.

RESULTS

**Logistic Regression Analysis**

We then set out to test whether the combination of these six interleukins and interleukin receptors will exhibit more prognostic value. With logistic regression we modelled the expression levels of the six interleukins to 10-year prostate cancer specific death after post-surgical salvage RT either with (IL&pGGG_model) or without (IL_model) the presence of the variable pathological Gleason grade group (pGGG). We tested the two models in ROC curve analysis as well as in Kaplan-Meier survival analysis. The logit(p) regression functions were derived as follows: IL_model:

$$c + (w_1 \cdot \mathrm{IL17RE}) + (w_2 \cdot \mathrm{IL1B}) + (w_3 \cdot \mathrm{IL3}) + (w_4 \cdot \mathrm{IL7R}) + (w_5 \cdot \mathrm{IL9R}) + (w_6 \cdot \mathrm{EBI3})$$

IL&pGGG model:

$$c + (w_1 \cdot \mathrm{IL17RE}) + (w_2 \cdot \mathrm{IL1B}) + (w_3 \cdot \mathrm{IL3}) + (w_4 \cdot \mathrm{IL7R}) + (w_5 \cdot \mathrm{IL9R}) + (w_6 \cdot \mathrm{EBI3}) + (w_7 \cdot \mathrm{pGGG})$$

The details for the weights $w_1$ to $w_7$ and the constant c are shown in the following TABLE 2.

TABLE 2: Variables and weights for the two logistic regression models, i.e., the Interleukin model (IL_model) and the Interleukin & pGGG combination model (IL&pGGG_model); NA - not available.

| Variable | Weights | | |
|---|---|---|---|
| Model | | IL_model | IL&pGGG_model |
| IL17RE | $w_1$ | 0.94141 | 1.88159 |
| IL1B | $w_2$ | -1.42739 | -0.041318 |

(continued)

| Variable | Weights | | |
|---|---|---|---|
| **Model** | | **IL_model** | **IL&pGGG_model** |
| **IL3** | $w_3$ | 1.26008 | 0.59493 |
| **IL7R** | $w_4$ | -1.91264 | -4.75981 |
| **IL9R** | $w_5$ | 0.50106 | -0.84648 |
| **EBI3** | $w_6$ | 5.10369 | 4.56796 |
| **pGGG** | $w_7$ | NA | 2.37674 |
| **Constant** | c | -5.174 | -7.60381 |

## ROC Curve Analysis

Next, we tested the logistic regression models as outlined above for their power to predict 10-year prostate cancer specific death after start of salvage radiation due to post-surgical disease recurrence. The performance of the models was compared to the clinical risk score CAPRA-S (see Cooperberg M.R. et al., "The CAPRA-S score: A straightforward tool for improved prediction of outcomes after radical prostatectomy", Cancer, Vol. 117, No. 22, pages 5039-5046, 2011).

Fig. 2 shows a ROC curve analysis of three predictive models. The IL_model (AUC=0,83) is the logistic regression model based on six interleukins. The IL&pGGG_model (AUC=0,92) is logistic regression model based on six interleukins and the pathology Gleason grade group (pGGG) information. The CAPRA_S (AUC=0,74) is the clinical CAPRA-S score (Cancer of the Prostate Risk Assessment score).

## Kaplan-Meier Survival Analysis

For Kaplan-Meier curve analysis, the logit(p) function of the two risk models (IL_model and IL&pGGG_model) was transferred into risk probabilities and the patient cohort was categorized into four sub-cohorts based on different arbitrarily selected cut-offs (see description of figures below). The goal was to create patient classes with a to some extent similar number of patients within the individual group. For better comparability, the same cut-offs were selected for both models.

The patient classes represent an increasing risk to experience the tested clinical endpoints of time to development of metastases (Figs. 3 and 4) or time to prostate cancer specific death (Figs. 5 and 6) since the start of salvage RT for the two created risk models (IL model; IL&pGGG_model).

Fig. 3 shows a Kaplan-Meier curve analysis of the IL_model. The clinical endpoint that was tested was the time to metastases (TTM) after the start of salvage radiation therapy (SRT) due to post-surgical disease recurrence. Patients were stratified into four groups according to their risk to experience the clinical endpoint as predicted by the respective logistic regression model. The following supplementary lists indicate the number of patients at risk for the IL_model classes analyzed, i.e., the patients at risk at any time interval +20 months after surgery are shown: Probability 0 to <0.1: 38, 35, 33, 32, 24, 15, 14, 4, 0, 0; Probability 0.1 to <0.25: 42, 38, 35, 34, 31, 26, 24, 11, 1, 0; Probability 0.25 to <0.5: 42, 31, 29, 27, 26, 23, 23, 14, 2, 0; Probability 0.5 to <1: 13, 7, 6, 5, 3, 3, 2, 0, 0, 0.

Fig. 4 shows a Kaplan-Meier curve analysis of the IL&pGGG_model. The clinical endpoint that was tested was the time to metastases (TTM) after the start of salvage radiation therapy (SRT) due to post-surgical disease recurrence. Patients were stratified into four groups according to their risk to experience the clinical endpoint as predicted by the respective logistic regression model. The following supplementary lists indicate the number of patients at risk for the IL&pGGG_model classes analyzed, i.e., the patients at risk at any time interval +20 months after surgery are shown: Probability 0 to <0.1: 48, 47, 47, 46, 40, 33, 32, 14, 0, 0; Probability 0.1 to <0.25: 30, 26, 26, 25, 22, 18, 16, 9, 2, 0; Probability 0.25 to <0.5: 19, 18, 16, 14, 13, 9, 8, 4,1, 9; Probability 0.5 to <1: 38, 20, 14, 13, 9, 7, 7, 2, 0, 0.

Fig. 5 shows a Kaplan-Meier curve analysis of the IL_model. The clinical endpoint that was tested was the time to prostate cancer specific death (PCa Death) after the start of the salvage radiation therapy (SRT) due to post-surgical disease recurrence. Patients were stratified into four groups according to their risk of experience the clinical endpoint as predicted by the respective logistic regression model. The following supplementary lists indicate the number of patients at risk for the IL_model classes analyzed, i.e., the patients at risk at any time interval +20 months after surgery are shown: Probability 0 to <0.1: 39, 39, 37, 36, 31, 22, 19, 9, 2, 2, 0; Probability 0.1 to <0.25: 43, 42, 39, 36, 34, 30, 25, 13, 1, 0, 0; Probability 0.25 to <0.5: 56, 52, 45, 35, 27, 23, 22, 13, 3, 1, 0; Probability 0.5 to <1: 13, 12, 9, 7, 4, 2, 1, 0, 0, 0, 0.

Fig. 6 shows a Kaplan-Meier curve of the IL&pGGG_model. The clinical endpoint that was tested was the time to prostate cancer specific death (PCa Death) after the start of salvage radiation therapy (SRT) due to post-surgical disease recurrence. Patients were stratified into four groups according to their risk to experience the clinical endpoint as predicted by the respective logistic regression model. The following supplementary lists indicate the number of patients at risk for the IL&pGGG_model classes analyzed, i.e., the patients at risk at any time interval +20 months after surgery are shown: Probability 0 to <0.1: 48, 48, 47, 47, 43, 37, 34, 17, 1, 1, 0; Probability 0.1 to <0.25: 32, 32, 30, 28, 24, 20, 15, 8, 3, 1, 0; Probability 0.25 to <0.5: 26, 26, 23, 20, 16, 13, 11, 6, 2, 1, 0; Probability 0.5 to <1: 45, 39, 30, 19, 13, 7, 7, 4, 0, 0, 0.

The Kaplan-Meier curve analysis as shown in Figs. 3 to 6 demonstrates the presence of different patient risk groups. The risk group of a patient is determined by the probability to suffer from the respective clinical endpoint (metastases, prostate cancer specific death) as calculated by the risk model IL_model or IL&pGGG model. Depending on the predicted risk of a patient (i.e., depending on in which risk group 1 to 4 the patient may belong) different types of interventions might be indicated. In the lowest risk groups (probability <0.25) standard of care (SOC), which is SRT potentially combined with SADT (salvage androgen deprivation therapy), delivers acceptable long-term oncological control. For the patient group with a risk between 0.25 and 0.5 dose escalation of the applied RT and/or combination with chemotherapy might provide improved longitudinal outcomes. This is definitely not the case for the patient group with a risk >0,5 to experience any of the relevant outcomes. In this patient group escalation of intervention is indicated. Options for escalation are early combination of SRT (considering higher dose regimens), SADT, and chemotherapy. Other options are alternative therapies like immunotherapies (e.g., Sipuleucil-T) or other experimental therapies.

**Discussion**

The effectiveness of both radical RT and SRT for localized prostate cancer is limited, resulting in disease progression and ultimately death of patients, especially for those at high risk of recurrence. The prediction of the therapy outcome is very complicated as many factors play a role in therapy effectiveness and disease recurrence. It is likely that important factors have not yet been identified, while the effect of others cannot be determined precisely. Multiple clinicopathological measures are currently investigated and applied in a clinical setting to improve response prediction and therapy selection, providing some degree of improvement. Nevertheless, a strong need remains for better prediction of the response to radical RT and to SRT, in order to increase the success rate of these therapies.

We have identified molecules of which expression shows a significant relation to mortality after radical RT and SRT and therefore are expected to improve the prediction of the effectiveness of these treatments. An improved prediction of effectiveness of RT for each patient be it in the radical or the salvage setting, will improve therapy selection and potentially survival. This can be achieved by 1) optimizing RT for those patients where RT is predicted to be effective (e.g. by dose escalation or a different starting time) and 2) guiding patients where RT is predicted not to be effective to an alternative, potentially more effective form of treatment. Further, this would reduce suffering for those patients who would be spared ineffective therapy and would reduce cost spent on ineffective therapies.

Other variations to the disclosed realizations can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

One or more steps of the method illustrated in Fig. 1 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the flowchart shown in Fig. 1, can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

The invention relates to a method of predicting a response of a prostate cancer subject to radiotherapy, comprising determining or receiving the result of a determination of a gene expression profile for each of one or more, for example, 1, 2, 3, 4, 5 or all, interleukin genes selected from the group consisting of: IL17RE, IL1B, IL3, IL7R, IL9R, and EBI3, said gene expression profile(s) being determined in a biological sample obtained from the subject, determining the prediction of the radiotherapy response based on the gene expression profile(s) for the one or more interleukin genes, and optionally, providing the prediction or a therapy recommendation based on the prediction to a medical caregiver or the subject. Since the status of the immune system and of the immune microenvironment have an impact on therapy effectiveness, the ability to identify markers predictive for this effect might help to be better able to predict overall RT response. Interleukins play a central role in the regulation of immune activity. The identified interleukins were found to exhibit a significant correlation with outcome after RT, wherefore we expect that they will provide predictive value with regard to the effectiveness of radical RT and/or SRT.

**The attached Sequence Listing, entitled 2019PF00710_Sequence Listing_ST25 is incorporated herein by reference, in its entirety.**

## Claims

**1.** A method of predicting a response of a prostate cancer subject to radiotherapy, comprising:

- determining or receiving the result of a determination of a gene expression profile for each of one or more, for example, 1, 2, 3, 4, 5 or all, interleukin genes selected from the group consisting of: IL17RE, IL1B, IL3, IL7R, IL9R, and EBI3, said gene expression profile(s) being determined in a biological sample obtained from the subject,
- determining the prediction of the radiotherapy response based on the gene expression profile(s) for the one or more interleukin genes, and
- optionally, providing the prediction or a therapy recommendation based on the prediction to a medical caregiver or the subject.

**2.** The method as defined in claim 1, wherein the one or more interleukin genes comprise three or more, preferably, all of the interleukin genes.

**3.** The method as defined in in claim 1 or 2, wherein the determining of the prediction of the radiotherapy response comprises combining the gene expression profiles for two or more, for example, 2, 3, 4, 5 or all, of the interleukin genes with a regression function that had been derived from a population of prostate cancer subjects.

**4.** The method as defined in claim 1 or 2, wherein the determining of the prediction of the radiotherapy response is further based on one or more clinical parameters obtained from the subject.

**5.** The method as defined in claim 4, wherein the one or more clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

**6.** The method as defined in claim 4 or 5, wherein the determining of the prediction of the radiotherapy response comprises combining the gene expression profile(s) for the one or more interleukin genes and the one or more clinical parameters obtained from the subject with a regression function that had been derived from a population of prostate cancer subjects.

**7.** The method as defined in any of claims 1 to 6, wherein the biological sample is obtained from the subject before the start of the radiotherapy.

**8.** The method as defined in any of claims 1 to 7, wherein the radiotherapy is radical radiotherapy or salvage radiotherapy.

**9.** The method as defined in any of claims 1 to 8, wherein the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

**10.** An apparatus for predicting a response of a prostate cancer subject to radiotherapy, comprising:

- an input adapted to receive data indicative of a gene expression profile for each of one or more, for example, 1, 2, 3, 4, 5 or all, interleukin genes selected from the group consisting of: IL17RE, IL1B, IL3, IL7R, IL9R, and EBI3, said gene expression profile(s) being determined in a biological sample obtained from the subject,

- a processor adapted to determine the prediction of the radiotherapy response based on the gene expression profile(s) for the one or more interleukin genes, and
- optionally, a providing unit adapted to provide the prediction or a therapy recommendation based on the prediction to a medical caregiver or the subject.

**11.** A computer program product comprising instructions which, when the program is executed by a computer, cause the computer to carry out a method comprising:

- receiving data indicative of a gene expression profile for each of one or more, for example, 1, 2, 3, 4, 5 or all, interleukin genes selected from the group consisting of: IL17RE, IL1B, IL3, IL7R, IL9R, and EBI3, said gene expression profile(s) being determined in a biological sample obtained from a prostate cancer subject,
- determining the prediction of the radiotherapy response based on the gene expression profile(s) for the one or more interleukin genes, and
- optionally, providing the prediction or a therapy recommendation based on the prediction to a medical caregiver or the subject.

**12.** A diagnostic kit, comprising:

- at least one primer and/or probe for determining the gene expression profile for each of one or more, for example, 1, 2, 3, 4, 5 or all, interleukin genes selected from the group consisting of: IL17RE, IL1B, IL3, IL7R, IL9R, and EBI3, in a biological sample obtained from the subject, and
- optionally, an apparatus as defined in claim 10 or a computer program product as defined in claim 11.

**13.** Use of the kit as defined in claim 12.

**14.** The use as defined in claim 13 in a method of predicting a response of a prostate cancer subject to radiotherapy.

**15.** A method, comprising:

- receiving a biological sample obtained from a prostate cancer subject,
- using the kit as defined in claim 12 to determine a gene expression profile for each of one or more, for example, 1, 2, 3, 4, 5 or all, interleukin genes selected from the group consisting of: IL17RE, IL1B, IL3, IL7R, IL9R, and EBI3, in the biological sample obtained from the subject.

**16.** Use of a gene expression profile for each of one or more, for example, 1, 2, 3, 4, 5 or all, interleukin genes selected from the group consisting of: IL17RE, IL1B, IL3, IL7R, IL9R, and EBI3, in a method of predicting a response of a prostate cancer subject to radiotherapy, comprising:

- determining the prediction of the radiotherapy response based on the gene expression profile(s) for the one or more interleukin genes, and
- optionally, providing the prediction or a therapy recommendation based on the prediction to a medical caregiver or the subject.

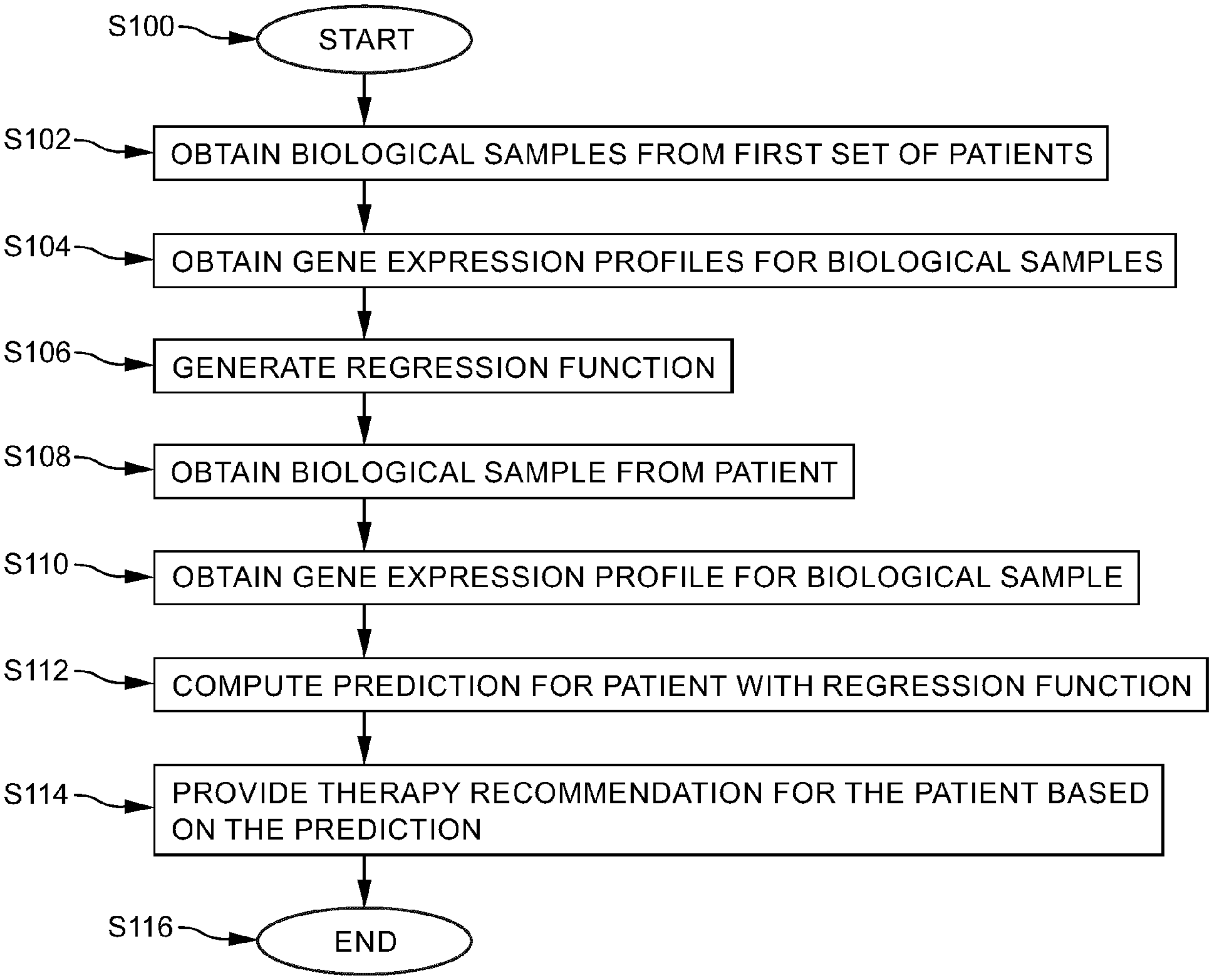
S100
START
S102
OBTAIN BIOLOGICAL SAMPLES FROM FIRST SET OF PATIENTS
S104
OBTAIN GENE EXPRESSION PROFILES FOR BIOLOGICAL SAMPLES
S106
GENERATE REGRESSION FUNCTION
S108
OBTAIN BIOLOGICAL SAMPLE FROM PATIENT
S110
OBTAIN GENE EXPRESSION PROFILE FOR BIOLOGICAL SAMPLE
S112
COMPUTE PREDICTION FOR PATIENT WITH REGRESSION FUNCTION
S114
PROVIDE THERAPY RECOMMENDATION FOR THE PATIENT BASED ON THE PREDICTION
S116
END

FIG. 1

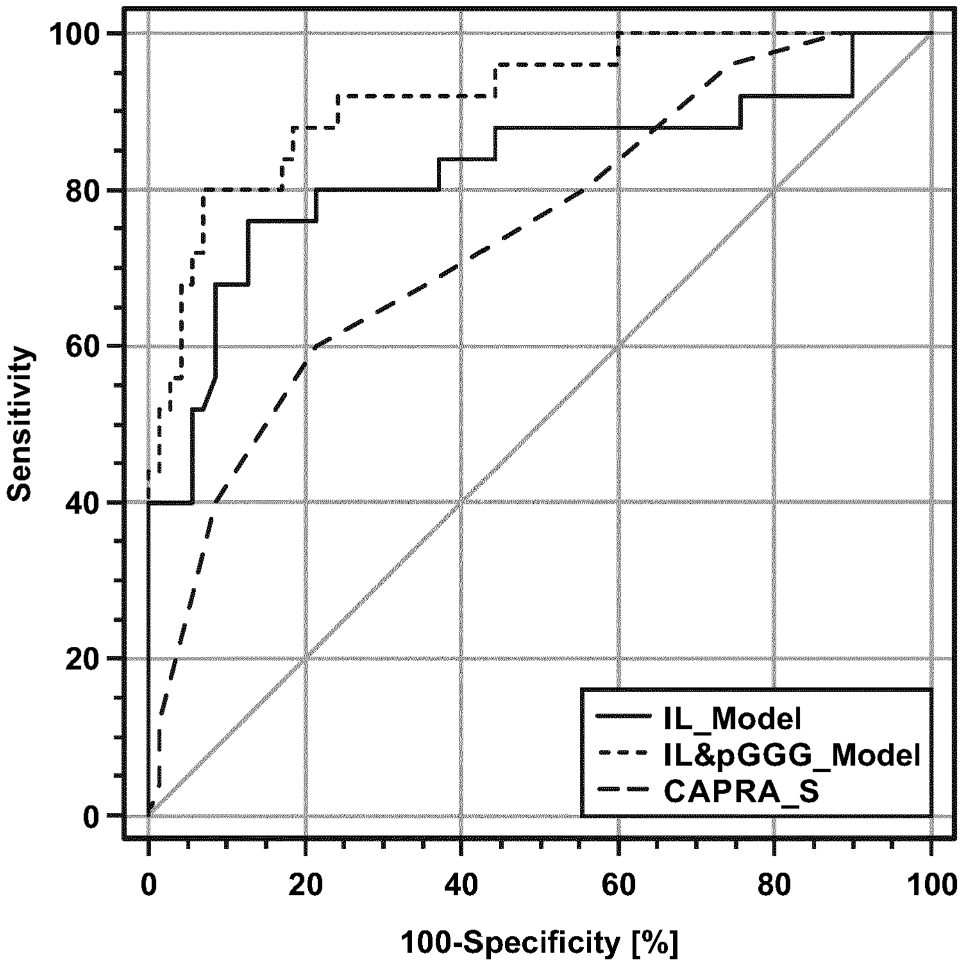
100
80
60
40
20
0
Sensitivity
0
20
40
60
80
100
100-Specificity [%]
IL_Model
IL&pGGG_Model
CAPRA_S

FIG. 2

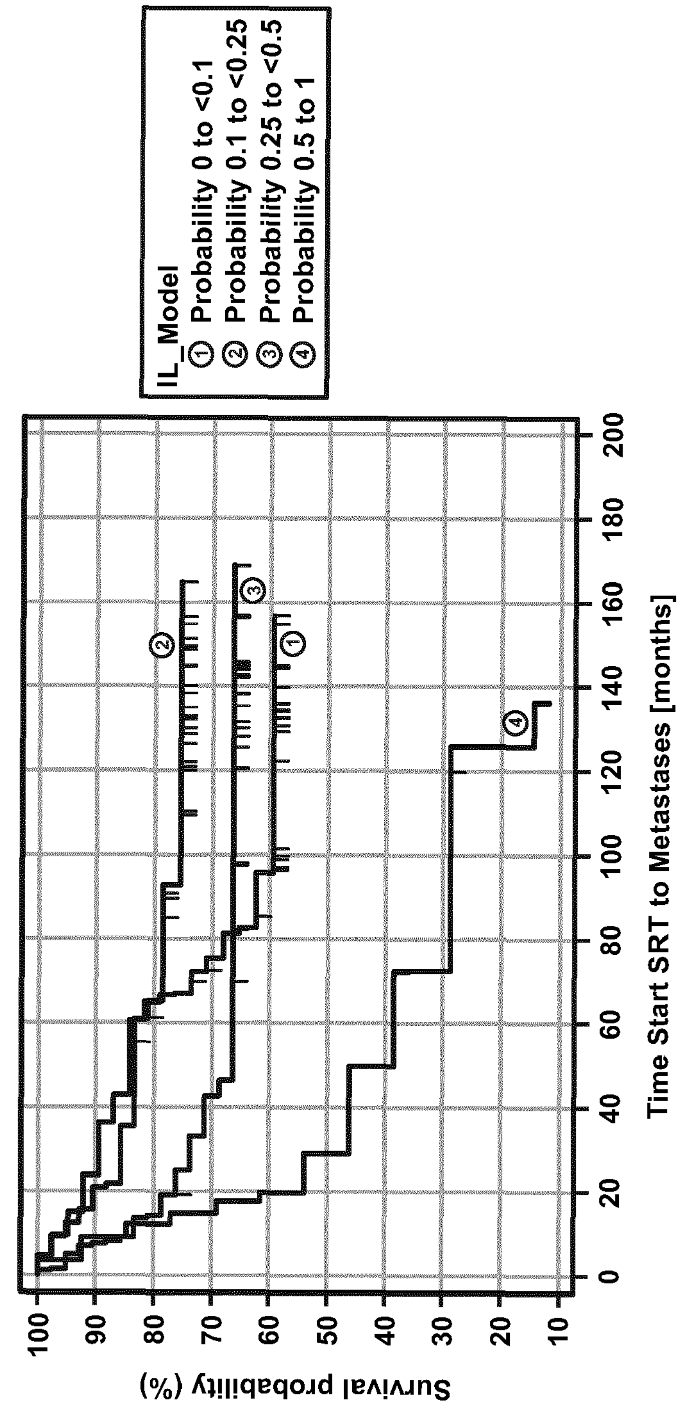
IL_Model
① Probability 0 to <0.1
② Probability 0.1 to <0.25
③ Probability 0.25 to <0.5
④ Probability 0.5 to 1
Survival probability (%)
100
90
80
70
60
50
40
30
20
10
0
20
40
60
80
100
120
140
160
180
200
Time Start SRT to Metastases [months]

FIG. 3

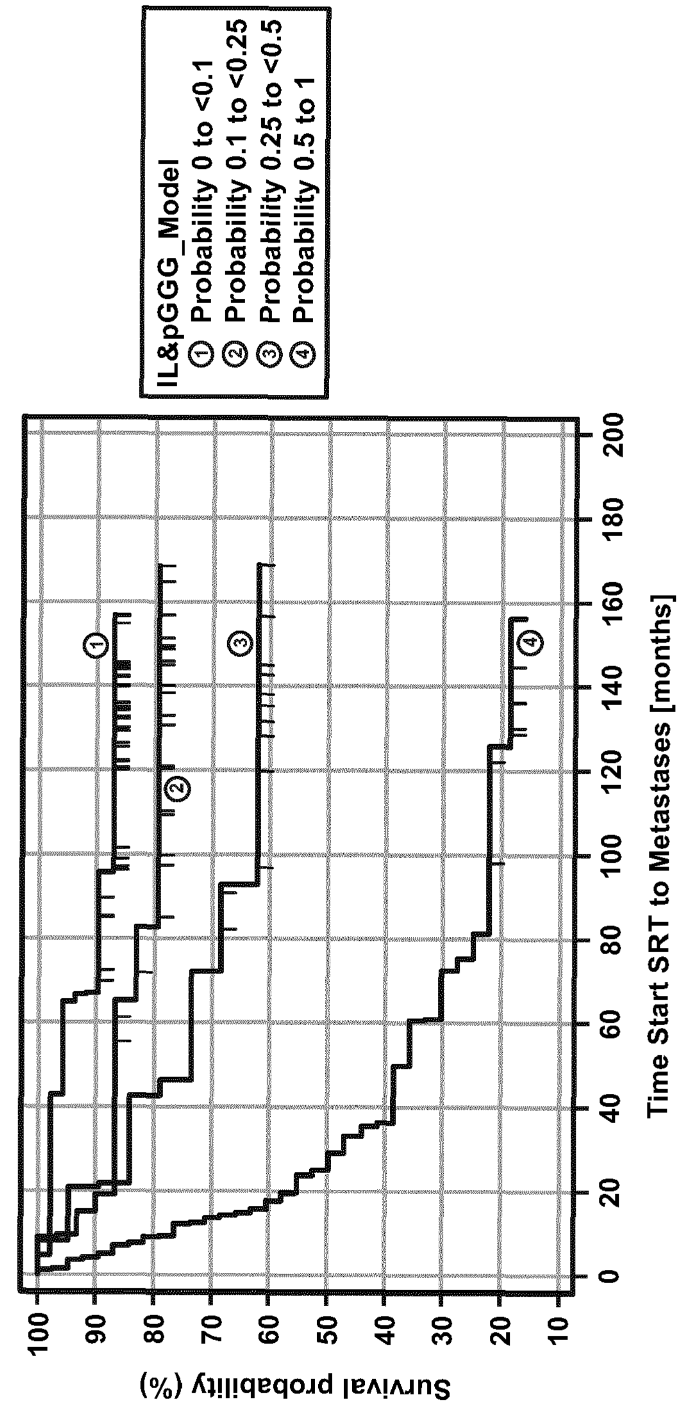
IL&pGGG_Model
① Probability 0 to <0.1
② Probability 0.1 to <0.25
③ Probability 0.25 to <0.5
④ Probability 0.5 to 1
Survival probability (%)
100
90
80
70
60
50
40
30
20
10
0
20
40
60
80
100
120
140
160
180
200
Time Start SRT to Metastases [months]
①
②
③
④

FIG. 4

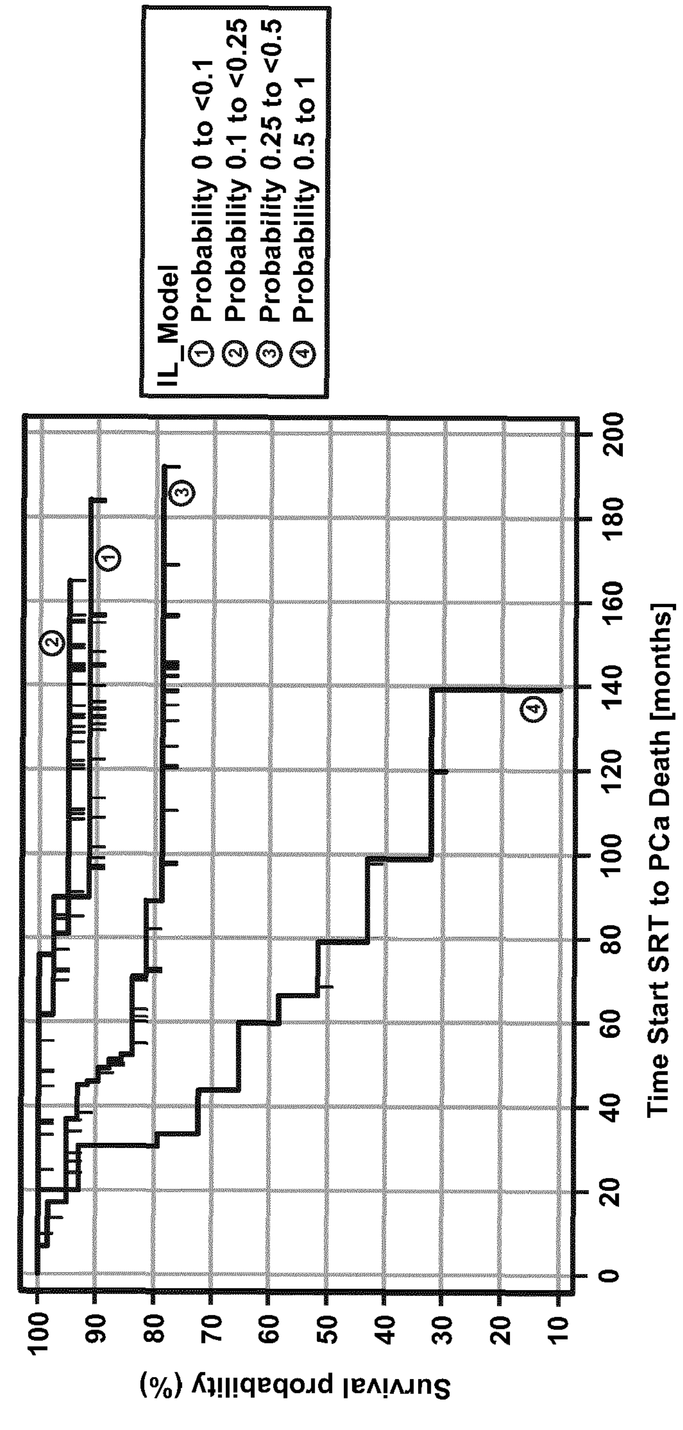
IL_Model
① Probability 0 to <0.1
② Probability 0.1 to <0.25
③ Probability 0.25 to <0.5
④ Probability 0.5 to 1
Survival probability (%)
100
90
80
70
60
50
40
30
20
10
0
20
40
60
80
100
120
140
160
180
200
Time Start SRT to PCa Death [months]
①
②
③
④

FIG. 5

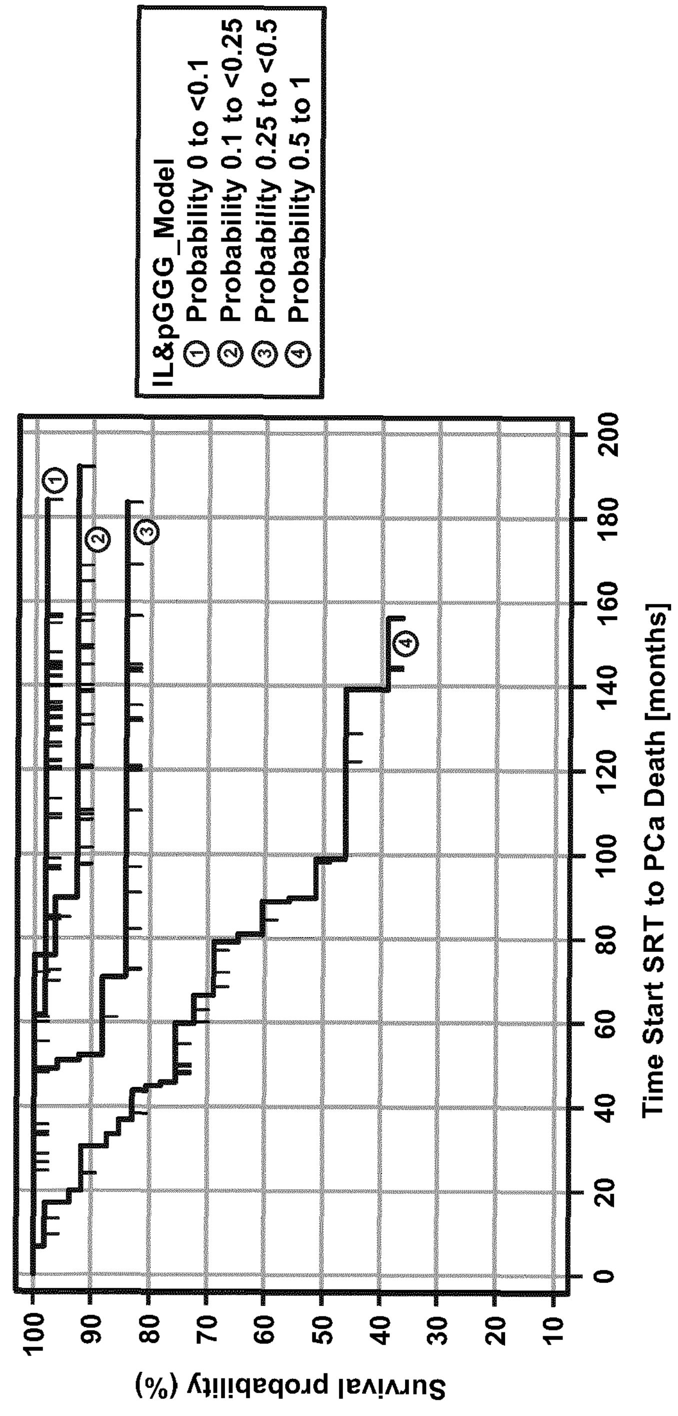
IL&pGGG_Model
① Probability 0 to <0.1
② Probability 0.1 to <0.25
③ Probability 0.25 to <0.5
④ Probability 0.5 to 1
Survival probability (%)
100
90
80
70
60
50
40
30
20
10
0
20
40
60
80
100
120
140
160
180
200
Time Start SRT to PCa Death [months]
①
②
③
④

FIG. 6

Europäisches Patentamt
European Patent Office
Office européen des brevets

# EUROPEAN SEARCH REPORT

Application Number
EP 20 16 1129

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/039490 A1 (GENOMEDX BIOSCIENCES INC [CA]; UNIV MICHIGAN REGENTS [US]) 1 March 2018 (2018-03-01) * claim 11; example 5 * ----- | 1-16 | INV. C12Q1/6886 |
| X | ZHAO SHUANG G ET AL: "Development and validation of a 24-gene predictor of response to postoperative radiotherapy in prostate cancer: a matched, retrospective analysis", THE LANCET ONCOLOGY, ELSEVIER, AMSTERDAM, NL, vol. 17, no. 11, 12 October 2016 (2016-10-12), pages 1612-1620, XP029794201, ISSN: 1470-2045, DOI: 10.1016/S1470-2045(16)30491-0 * the whole document * ----- | 1-16 | |

TECHNICAL FIELDS SEARCHED (IPC)

C12Q

The present search report has been drawn up for all claims

1

| Place of search | Date of completion of the search | Examiner |
|---|---|---|
| The Hague | 28 August 2020 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 16 1129

This annex lists the patent family members relating to the patent documents cited in the above-mentioned European search report. The members are as contained in the European Patent Office EDP file on
The European Patent Office is in no way liable for these particulars which are merely given for the purpose of information.

28-08-2020

| Patent document cited in search report | Publication date | Patent family member(s) | Publication date |
|---|---|---|---|
| WO 2018039490 A1 | 01-03-2018 | AU 2017315425 A1 | 18-04-2019 |
| | | CN 110506127 A | 26-11-2019 |
| | | EP 3504348 A1 | 03-07-2019 |
| | | US 2019218621 A1 | 18-07-2019 |
| | | WO 2018039490 A1 | 01-03-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BRAY F. et al.** Global cancer statistics 2018: GLOBOCAN estimates of incidence and mortality worldwide for 36 cancers in 185 countries. *CA Cancer J Clin,* 2018, vol. 68 (6), 394-424
- Cancer Facts & Figures 2010. ACS (American Cancer Society), 2010
- **GRIMM P. et al.** Comparative analysis of prostate-specific antigen free survival outcomes for patients with low, intermediate and high risk prostate cancer treatment by radical therapy. Results from the Prostate Cancer Results Study Group. *BJU Int,* 2012, 22-29
- **DAL PRA A. et al.** Contemporary role of postoperative radiotherapy for prostate cancer. *Transl Androl Urol,* 2018, vol. 7 (3), 399-413
- **HERRERA F.G. ; BERTHOLD D.R.** Radiation therapy after radical prostatectomy: Implications for clinicians. *Front Oncol,* 2016, vol. 6 (117
- **PISANSKY T.M. et al.** Salvage radiation therapy dose response for biochemical failure of prostate cancer after prostatectomy - A multi-institutional observational study. *Int J Radiat Oncol Biol Phys,* 2016, vol. 96 (5), 1046-1053
- **RESNICK M.J. et al.** Long-term functional outcomes after treatment for localized prostate cancer. *N Engl J Med,* 2013, vol. 368 (5), 436-445
- **HEGARTY S.E. et al.** Radiation therapy after radical prostatectomy for prostate cancer: Evaluation of complications and influence of radiation timing on outcomes in a large, population-based cohort. *PLoS One,* 2015, vol. 10 (2
- **PARAVATI A.J. et al.** Variation in the cost of radiation therapy among medicare patients with cancer. *J Oncol Pract,* 2015, vol. 11 (5), 403-409
- **HALL W.A. et al.** Biomarkers of outcome in patients with localized prostate cancer treated with radiotherapy. *Semin Radiat Oncol,* 2016, vol. 27, 11-20
- **RAYMOND E. et al.** An appraisal of analytical tools used in predicting clinical outcomes following radiation therapy treatment of men with prostate cancer: A systematic review. *Radiat Oncol,* 2017, vol. 12 (1), 56
- **MANTOVANI A. et al.** Cancer-related inflammation. *Nature,* 2008, vol. 454 (7203), 436-444
- **GIRALDO N.A. et al.** The clinical role of the TME in solid cancer. *Br J Cancer,* 2019, vol. 120 (1), 45-53
- **SHIAO S.L. et al.** Regulation of prostate cancer progression by tumor microenvironment. *Cancer Lett,* 2016, vol. 380 (1), 340-348
- **BARKER H.E. et al.** The tumor microenvironment after radiotherapy: Mechanisms of resistance or recurrence. *Nat Rev Cancer,* 2015, vol. 15 (7), 409-425
- **BROCKER C. et al.** Evolutionary divergence and functions of the human interleukin (IL) gene family. *Hum Genomics,* 2010, vol. 5 (1), 30-55
- **HALL W.A. et al.** The influence of the pretreatment host immune inflammatory state and response to radiation therapy in high-risk adenocarcinoma of the prostate. *A validation study from NRG Oncology/RTOG 0521,* 2018, vol. 102 (3), S13-S14
- **COOPERBERG M.R. et al.** The CAPRA-S score: A straightforward tool for improved prediction of outcomes after radical prostatectomy. *Cancer,* 2011, vol. 117 (22), 5039-5046